(19) Europäisches Patentamt
European Patent Office
Office européen des brevets

(11) **EP 1 207 884 B1**

(12) **EUROPÄISCHE PATENTSCHRIFT**

(45) Veröffentlichungstag und Bekanntmachung des Hinweises auf die Patenterteilung:
**03.11.2004 Patentblatt 2004/45**

(51) Int Cl.⁷: **A61K 31/495**, A61K 31/445, A61K 31/505, A61K 31/55, A61K 31/54, A61P 15/12

(21) Anmeldenummer: 00958385.7

(22) Anmeldetag: **05.08.2000**

(86) Internationale Anmeldenummer:
**PCT/EP2000/007613**

(87) Internationale Veröffentlichungsnummer:
**WO 2001/010425 (15.02.2001 Gazette 2001/07)**

(54) **VERWENDUNG VON CGRP-ANTAGONISTEN UND CGRP-RELEASE-HEMMERN ZUR BEKÄMPFUNG MENOPAUSALER HITZEWALLUNGEN**

USE OF CGRP ANTAGONISTS AND CGRP RELEASE INHIBITORS FOR CONTROLLING MENOPAUSAL HOT FLASHES

UTILISATION D'ANTAGONISTES DE CGRP ET D'INHIBITEURS DE SECRETION CGRP SERVANT A LUTTER CONTRE LES BOUFFEES DE CHALEUR DE LA MENOPAUSE

(84) Benannte Vertragsstaaten:
**AT BE CH CY DE DK ES FI FR GB GR IE IT LI LU MC NL PT SE**
Benannte Erstreckungsstaaten:
**LT LV RO SI**

(30) Priorität: **10.08.1999 DE 19937304**

(43) Veröffentlichungstag der Anmeldung:
**29.05.2002 Patentblatt 2002/22**

(73) Patentinhaber: **Boehringer Ingelheim Pharma GmbH & Co.KG**
**55218 Ingelheim am Rhein (DE)**

(72) Erfinder:
• **DOODS, Henri**
**88447 Warthausen (DE)**
• **RUDOLF, Klaus**
**88447 Warthausen (DE)**
• **EBERLEIN, Wolfgang**
**88400 Biberach (DE)**
• **ENGEL, Wolfhard**
**88400 Biberach (DE)**

(56) Entgegenhaltungen:
EP-A- 0 821 061          WO-A-98/11128
DE-A- 19 911 039         US-A- 5 910 482

• DOODS: "pharmacological profile..." BR. J. PHARMACOL., Bd. 129, Nr. 3, 2000, Seiten 420-423, XP000992559

**Beschreibung**

Hitzewallungen sind ein verbreitetes Symptom des peri/postmenopausalen Syndroms, dessen Physiologie bis heute nicht vollständig verstanden ist. Außer der Hormonsubstitution (hormone replacement therapy), die eine komplexe Intervention darstellt und aufgrund der damit verbundenen Nebenwirkungen häufig nicht dauerhaft angewandt werden kann, existiert bislang keine einfach durchzuführende, nebenwirkungsarme Therapie für diese im allgemeinen als lästig empfundene Erscheinung.

Hitzewallungen werden durch Gefäßerweiterung und erhöhten Blutfluß verursacht. Bereits mehrfach wurde die Vermutung publiziert, daß CGRP (calcitonin gene-related peptide) aufgrund der vasodilatorischen Eigenschaften dieses Neuropeptids eine Rolle bei der Entstehung menopausaler Hitzewallungen östrogendefizienter Frauen spielt ([1]: J. Endocrinol. (1995), 146(3), 431-437; [2]: Acta Physiol. Scand. (1998), 162(4), 517-522; [3]: Am. J. Obstet. Gynecol. (1996), 175(3, Pt. 1), 638-642). Ein therapeutischer Einsatz von CGRP-Antagonisten zur Behandlung des menopausalen Syndroms wurde in der Literatur noch nicht vorgeschlagen.

Es wurde nun gefunden, daß die Symptomatik menopausaler Hitzewallungen durch Substanzen, die die Wirkungen von CGRP antagonisieren (CGRP-Antagonisten) oder die Freisetzung von CGRP aus sensorischen Nervenendigungen hemmen oder vermindern (CGRP-Release-Hemmer), effektiv verhindert oder in ihrer beeinträchtigenden Wirkung erheblich abgeschwächt werden kann, wobei sich dieser Therapieansatz insbesondere durch die Nebenwirkungsarmut vor der Hormonsubstitution auszeichnet.

Gegenstand der vorliegenden Erfindung ist somit die Verwendung von CGRP-Antagonisten oder/und CGRP-Release-Hemmern zur Bekämpfung menopausaler Hitzewallungen, wobei sowohl die Prävention als auch die Akut-Behandlung eingeschlossen sind. Die erfindungsgemäße Verwendung betrifft bevorzugt die Monotherapie mit einer Einzelsubstanz, schließt jedoch auch die Kombinationstherapie mit mehreren Substanzen der genannten Wirkstoffgruppen ein. Ferner kann die erfindungsgemäße Verwendung in Ergänzung zu einer üblicherweise durchgeführten Hormonsubstitution erfolgen.

Von den in der WO 98/11128 beschriebenen CGRP-Antagonisten kommen zur Bekämpfung menopausaler Hitzewallungen, zur Herstellung eines entsprechenden Arzneimittels sowie als Bestandteil eines entsprechenden Arzneimittels vorzugsweise folgende Verbindungen in Frage:

(A) 1-[N$^2$-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,

(B) 1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,

(C) 1-[N$^2$-[4-Amino-3,5-dibrom-N-[[4-(3,4-dihydro-2(1H)-oxo-chinazolin-3-yl)-1-piperidinyl]carbonyl]-D-phenylalanyl]-L-lysyl]-4-(4-pyridinyl)-piperazin,

(D) 1-[N$^2$-[4-Amino-3,5-dibrom-N-[[4-(3,4-dihydro-2(1H)-oxochinazolin-3-yl)-1-piperidinyl]carbonyl]-D-phenylalanyl]-L-lysyl]-4-(4-pyridinyl)-piperidin,

(E) 1-[N$^2$-[3,5-Dibrom-N-[[4-(1,3-dihydro-4-phenyl-2(2H)-oxoimidazol-1-yl)-1-piperidinyl]carbonyl]-D-tyrosyl]-L-lysyl]-4-(4-pyridinyl)-piperazin,

(F) 1-[N$^2$-[4-Amino-3,5-dibrom-N-[[4-(1,3-dihydro-4-phenyl-2(2H)-oxoimidazol-1-yl)-1-piperidinyl]carbonyl]-D-phenylalanyl]-L-lysyl]-4-(4-pyridinyl)-piperazin,

(G) 1-[3,5-Dibrom-N-[[4-(3,4-dihydro-2(1H)-oxothieno[3,4-d]-pyrimidin-3-yl)-1-piperidinyl]carbonyl]-D-tyrosyl]-4-(1-piperidinyl)-piperidin,

(H) 1-[4-Amino-3,5-dibrom-N-[[4-(2,4-dihydro-5-phenyl-3(3H)-oxo-2,2,4-triazol-2-yl)-1-piperidinyl]carbonyl]-D-phenylalanyl]-4-(1-methyl-4-piperidinyl)-piperidin,

(I) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-piperidinyl)-piperidin,

(J) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazin,

(K)   1-[4-Amino-3,5-dibrom-N-[[4-(3,4-dihydro-2(1H)-oxothieno[3,   2-d]pyrimidin-3-yl)-1-piperidinyl]carbonyl]-D-phenylalanyl]-4-(1-piperidinyl)-piperidin,

(L)   1-[4-Amino-3,5-dibrom-N-[[4-[1,3-dihydro-4-[3-(trifluoromethy)phenyl]-2(2H)-oxoimidazol-1-yl]-1-piperidinyl] carbonyl]-D-phenylalanyl]-4-(1-ethyl-4-piperidinyl)-piperidin,

(M)   1-[4-Amino-3,5-dibrom-N-[[4-[3,4-dihydro-2(1H)-oxochinazolin-3-yl]-1-piperidinyl]carbonyl]-D-phenylalanyl]-4-(1-hexyl-4-piperidinyl)-piperidin,

(N)   1-[4-Amino-3,5-dibrom-N-[[4-[3,4-dihydro-2(1H)-oxochinazolin-3-yl]-1-piperidinyl]carbonyl]-D-phenylalanyl]-4-(1-cyclopropylmethyl-4-piperidinyl)-piperidin,

(O)   1-[N-[[4-[3,4-Dihydro-2(1H)-oxochinazolin-3-yl]-1-piperidinyl]carbonyl]-3-ethenyl-D,L-phenylalanyl]-4-(hexahydro-1H-1-azepinyl)-piperidin,

(P)   (R,S)-1-[4-[4-(3,4-Dihydro-2(1H)-oxochinazolin-3-yl)-1-piperidinyl]-2-[(4-hydroxy-3,5-dimethylphenyl)methyl]-1,4-dioxobutyl]-4-(1-piperidinyl)-piperidin,

(Q)   1-[4-Amino-3,5-dibrom-N-[[4-[N-(aminocarbonyl)-N-phenylamino]-1-piperidinyl]carbonyl]-D-phenylalanyl]-4-(1-piperidinyl)-piperidin,

(R)   1-[4-Amino-3,5-dibrom-N-[[4-(3,4-dihydro-2(1H)-oxochinazolin-3-yl)-1-piperidinyl]carbonyl]-D-phenylalanyl]-4-(5-methoxy-4-pyrimidinyl)-piperazin,

(S)   1-[4-Amino-3,5-dibrom-N-[[4-(1,1-dioxido-3(4H)-oxo-1,2,4-benzothiadiazin-2-yl)-1-piperidinyl]carbonyl]-D-phenylalanyl]-4-(1-piperidinyl)-piperidin,

(T)   1-[4-Amino-3,5-dibrom-N-[[4-[2(1H)-oxochinolin-3-yl]-1-piperidinyl]carbonyl]-D-phenylalanyl]-4-(1-piperidinyl)-piperidin,

(U)   1-[4-Amino-3,5-dibrom-N-[[4-[3,4-dihydro-2(1H)-oxochinazolin-3-yl]-1-piperidinyl]carbonyl]-D-phenylalanyl]-4-[3-(dimethylamino)propyl]-piperazin,

(V)   1-E4-Amino-3,5-dibrom-N-[[4-[3,4-dihydro-2(1H)-oxochinazolin-3-yl]-1-piperidinyl]carbonyl]-D-phenylalanyl]-4-(4-methyl-1-piperazinyl)-piperidin,

(W)   1-[4-Amino-3,5-dibrom-N-[[4-[3,4-dihydro-2(1H)-oxochinazolin-3-yl]-1-piperidinyl]carbonyl]-D-phenylalanyl]-4-[(1-methyl-4-piperidinyl)carbonyl]-piperazin,

(X)   1-[4-Amino-3,5-dibrom-N-[[4-(3,4-dihydro-2(1H)-oxochinazolin-3-yl)-1-piperidinyl]carbonyl]-D-phenylalanyl]-4-[(1-methyl-4-piperazinyl)carbonyl]-piperazin,

(Y)   1-[4-Amino-3,5-dibrom-N-[[4-(3,4-dihydro-2(1H)-oxochinazolin-3-yl)-1-piperidinyl]carbonyl]-D-phenylalanyl]-4-[4-[4-(dimethylamino)butyl]phenyl]-piperazin,

(Z)   1-[4-Amino-3,5-dibrom-N-[[4-(3,4-dihydro-2(1H)-oxochinazolin-3-yl)-1-piperidinyl]carbonyl]-D-phenylalanyl]-4-[4-(dimethylamino)-1-piperidinyl]-piperidin,

(AA)1-[N$^2$-[[4-(1,3-Dihydro-4-phenyl-2(2H)-oxoimidazol-1-yl)-1-piperidinyl]carbonyl]-N'-methyl-D-tryptyl]-4-(4-methyl-1-piperazinyl)-piperidin,

(AB)1-[N$^2$-[[4-(1,3-Dihydro-4-phenyl-2(2H)-oxoimidazol-1-yl)-1-piperidinyl]carbonyl]-N'-(1,1-dimethylethoxycarbonyl)-Dtryptyl]-4-(1-methyl-4-piperidinyl)-piperidin,

(AC)   (R,S)-1-[4-[4-(3,4-Dihydro-2(1H)-oxochinazolin-3-yl)-1-piperidinyl]-2-[(3,5-dibrom-4-methylphenyl)methyl]-1,4-dioxobutyl]-4-(4-methyl-1-piperazinyl)-piperidin,

(AD)   (R,S)-1-[4-[4-(3,4-Dihydro-2(1H)-oxochinazolin-3-yl)-1-piperidinyl]-2-[(3,5-dibrom-4-methoxyphenyl)methyl]-1,4-dioxobutyl]-4-(1-methyl-4-piperidinyl)-piperidin,

(AE)  (R,S)-1-[4-(4-(3,4-Dihydro-2(1H)-oxochinazolin-3-yl)-1-piperidinyl]-2-[(3,4-dibromphenyl)methyl]-1,4-dioxobutyl]-4-(4-methyl-1-piperazinyl)-piperidin,

(AF) 1-[N²-[N-[[4-(1,3-Dihydro-2(2H)-oxobenzimidazol-1-yl)-1-piperidinyl]carbonyl]-3,5-dibrom-D-tyrosyl]-L-lysyl]-4-(4-pyridinyl)-piperazin,

(AG)    1-[4-Amino-3,5-dibrom-N-[[4-(1,3-dihydro-6-hydroxy-2(2H)-oxobenzimidazol-1-yl)-1-piperidinyl]carbonyl]-D-phenylalanyl]-4-(1-piperidinyl)-piperidin,

(AH)   1-[N²-[4-Amino-3,5-dibrom-N-[[4-(1,3-dihydro-2(2H)-oxo-benzimidazol-1-yl)-1-piperidinyl]carbonyl]-D-phenylalanyl]-N⁶,N⁶-dimethyl-L-lysyl]-4-(4-pyridinyl)-piperazin,

(AI)  1-[N²-[4-Amino-3,5-dibrom-N-[[4-(3,4-dihydro-2(1H)-oxochinazolin-3-yl)-1-piperidinyl]carbonyl]-D-phenylalanyl]-N⁶,N⁶-dimethyl-L-lysyl]-4-(4-pyridinyl)-piperazin,

(AJ)  (R,S)-1-[2-(4-Amino-3,5-dibrombenzoyl)-4-[4-(3,4-dihydro-2(1H)-oxochinazolin-3-yl)-1-piperidinyl]-4-oxobutyl]-4-(1-piperidinyl)-piperidin,

(AK)  1-[4-Amino-3,5-dibrom-N-[[4-(3,4-dihydro-2,2-dioxido-2,1,3-benzothiadiazin-3-yl)-1-piperidinyl]carbonyl]-D-phenylalanyl]-4-(1-piperidinyl)-piperidin,

(AL)    1-[4-Amino-3,5-dibrom-N-[[4-[1,3-dihydro-2(2H)-oxoimidazo[4,5-c]chinolin-3-yl]-1-piperidinyl]carbonyl]-D-phenylalanyl]-4-(1-piperidinyl)carbonyl]-piperidin,

(AM)    1-[4-Amino-3,5-dibrom-N-[[4-(3,4-dihydro-2(1H)-oxochinazolin-3-yl)-1-piperidinyl]carbonyl]-D-phenylalanyl]-4-(1-piperidinyl)-piperidin,

(AN)   1-[N²-[3,5-Dibrom-N-[[4-(3,4-dihydro-2(1H)-oxochinazolin-3-yl)-1-piperidinyl]carbonyl]-D-tyrosyl]-N⁶,N⁶-dimethyl-L-lysyl]-4-(4-pyridinyl)-piperazin,

(AO) 1-[4-Amino-N-[[4-[4-(3-bromphenyl)-1,3-dihydro-2(2H)-oxoimidazol-1-yl]-1-piperidinyl]carbonyl]-3,5-dibrom-D-phenylalanyl]-4-(1-methyl-4-piperidinyl)-piperidin,

(AP)    1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(4-methyl-1-piperazinyl)-piperidin,

(AQ)   1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-piperidinyl)-piperidin,

(AR)  1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(exo-8-methyl-8-azabicyclo[3,2,1]oct-3-yl)-piperazin,

(AS)      1-[3,5-Dibrom-N-[[4-(1,3-dihydro-4-phenyl-2(2H)-oxoimidazol-1-yl)-1-piperidinyl]carbonyl]-D-tyrosyl]-4-(1-piperidinyl)-piperidin,

(AT)  1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-ethyl-4-piperidinyl)-piperazin,

(AU)   1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(hexahydro-4-methyl-1H-1,4-diazepin-1-yl)piperidin,

(AV)1-[3,5-Dibrom-N-[[4-(3,4-dihydro-2(1H)-oxochinazolin-3-yl)-1-piperidinyl]carbonyl]-D-tyrosyl]-4-[1-(methylsulfonyl)-4-piperidinyl]-piperidin,

(AW)  1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperidin,

(AX) 1-[3,5-Dibrom-N-[[4-[1,3-dihydro-4-[3-(trifluormethyl)-phenyl]-2(2H)-oxoimidazol-1-yl]-1-piperidinyl]carbonyl]-D-tyrosyl]-4-(hexahydro-1H-1-azepinyl)-piperidin,

(AY) 1-[3,5-Dibrom-N-[[4-(3,4-dihydro-2(1H)-oxochinazolin-3-yl)-1-piperidinyl]carbonyl]-D-tyrosyl]-4-(1-methyl-4-piperidinyl)-piperidin,

(AZ) 1-[4-Amino-3,5-dibrom-N-[[4-(3,4-dihydro-2(1H)-oxochinazolin-3-yl)-1-piperidinyl]carbonyl]-D-phenylalanyl]-4-(exo-8-methyl-8-azabicyclo[3,2,1]oct-3-yl)-piperazin,

(BA) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperazin,

(BB) 1-[3,5-Dibrom-N-[[4-[1,3-dihydro-4-[3-(trifluormethyl)-phenyl]-2(2H)-oxoimidazol-1-yl]-1-piperidinyl]carbonyl]-D-tyrosyl]-4-(1-piperidinyl)-piperidin,

(BC) 1-[N$^6$-Acetyl-N$^2$-[3,5-dibrom-N-[[4-(3,4-dihydro-2(1H)-oxochinazolin-3-yl)-1-piperidinyl]carbonyl]-D-tyrosyl]-Llysyl]-4-(4-pyridinyl)-piperazin,

(BD) 1-[3,5-Dibrom-N-[[4-(1,3-dihydro-4-phenyl-2(2H)-oxoimidazol-1-yl)-1-piperidinyl]carbonyl]-D-tyrosyl]-4-(hexahydro-1H-1-azepinyl)-piperidin,

(BE) 1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperidin,

(BF) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-methyl-4-piperidinyl)-piperidin,

(BG) 1-[3,5-Dibrom-N-[[4-(3,4-dihydro-2(1H)-oxochinazolin-3-yl)-1-piperidinyl]carbonyl]-D-tyrosyl]-4-[1-(hydroxycarbonylmethyl)-4-piperidinyl]-piperidin,

(BH) 1-[4-Amino-3,5-dibrom-N-[[4-[3,4-dihydro-2(1H)-oxochinazolin-3-yl]-1-piperidinyl]carbonyl]-D-phenylalanyl]-4-(1-methylsulfonyl-4-piperidinyl)-piperidin,

(BI) 1-[3,5-Dibrom-N-[[4-(3,4-dihydro-2(1H)-oxochinazolin-3-yl)-1-piperidinyl]carbonyl]-D-tyrosyl]-4-(4-piperidinyl)-piperidin,

(BJ) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-ethyl-4-piperidinyl)-piperidin,

(BK) 1-[4-Amino-3,5-dibrom-N-[[4-(1,3-dihydro-4-(3-hydroxyphenyl)-2(2H)-oxoimidazol-1-yl)-1-piperidinyl]carbonyl]-D-phenylalanyl]-4-(1-methyl-4-piperidinyl)-piperidin,

(BL) 1-[3,5-Dibrom-N-[[4-(3,4-dihydro-2(1H)-oxochinazolin-3-yl)-1-piperidinyl]carbonyl]-D-tyrosyl]-4-(hexahydro-1H-1-azepinyl)-piperidin,

(BM) 1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-piperidinyl)-piperidin,

(BN) 1-[4-Amino-3,5-dibrom-N-[[4-[4-(3-bromphenyl)-1,3-dihydro-2(2H)-oxoimidazol-1-y]-1-piperidinyl]carbonyl]-D-phenylalanyl]-4-(exo-8-methyl-8-azabicyclo[3,2,1]oct-3-yl)-piperazin,

(BO) 1-[4-Amino-3,5-Dibrom-N-[[4-(3,4-dihydro-2(1H)-oxo-chinazolin-3-yl)-1-piperidinyl]carbonyl]-D-phenylalanyl]-4-(1-ethyl-4-piperidinyl)-piperidin,

(BP) 1-[4-Amino-3,5-Dibrom-N-[[4-(3,4-dihydro-2(1H)-oxochinazolin-3-yl)-1-piperidinyl]carbonyl]-D-phenylalanyl]-4-(1-ethyl-4-piperidinyl)-piperazin,

(BQ) 1-[4-Amino-3,5-dibrom-N-[[4-[1,3-dihydro-4-(3-methoxyphenyl)-2(2H)-oxoimidazol-1-yl]-1-piperidinyl]carbonyl]-D-phenylalanyl]-4-(exo-8-methyl-8-azabicyclo[3,2,1]oct-3-yl)-piperazin,

(BR) 1-[3,5-Dibrom-N-[[4-(3,4-dihydro-2(1H)-oxochinazolin-3-yl)-1-piperidinyl]carbonyl]-D-tyrosyl]-4-[1-(cyclopropylmethyl)-4-piperidinyl]-piperidin,

(BS)   1-[4-Amino-3,5-Dibrom-N-[[4-(3,4-dihydro-2(1H)-oxochinazolin-3-yl)-1-piperidinyl]carbonyl]-D-phenylalanyl]-4-(hexahydro-1H-1-azepinyl)-piperidin.

(BT) 1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin,

(BU)    1-[3,5-Dibrom-N-[[4-(1,3-dihydro-4-phenyl-2(2H)-oxoimidazol-1-yl)-1-piperidinyl]carbonyl]-D-tyrosyl]-4-(4-pyridinyl)-piperidin,

(BV) 1-[3,5-Dibrom-N-[[4-[1,3-dihydro-4-[3-(trifluormethyl)-phenyl]-2(2H)-oxoimidazol-1-yl]-1-piperidinyl]carbonyl]-D-tyrosyl]-4-(1-methyl-4-piperidinyl)-piperazin,

(BW)  1-[N2-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-L-lysyl]-4-(4-pyridinyl)-piperazin,

(BX)    1-[3,5-Dibrom-N-[[4-(1,3-dihydro-4-(3-thienyl)-2(2H)-oxoimidazol-1-yl)-1-piperidinyl]carbonyl]-D-tyrosyl]-4-(1-piperidinyl)-piperidin,

(BY) 1-[4-Amino-N-[[4-(4-(3-chlorphenyl)-1,3-dihydro-2(2H)-oxoimidazol-1-yl)-1-piperidinyl]carbonyl]-3,5-dibrom-D-phenylalanyl]-4-(1-methyl-4-piperidinyl)-piperidin,

(BZ)   1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(hexahydro-1H-1-azepinyl)-piperidin,

(CA)  1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-methyl-4-piperidinyl)-piperazin,

(CB) 1-[4-Amino-N-[[4-4-(3-chlorphenyl)-1,3-dihydro-2(2H)-oxoimidazol-1-yl]-1-piperidinyl]carbonyl]-3,5-dibrom-D-phenylalanyl]-4-(hexahydro-1H-1-azepinyl)-piperidin,

(CC)   1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(4-pyridinyl)-piperazin,

(CD)    1-[3,5-Dibrom-N-[[4-(1,3-dihydro-4-phenyl-2(2H)-oxoimidazol-1-yl)-1-piperidinyl]carbonyl]-D-tyrosyl]-4-(1-methyl-4-piperidinyl)-piperidin,

(CE)    1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-[4-(1-oxoethyl)phenyl]-piperazin,

(CF)    1-[3,5-Dibrom-N-[[4-[3,4-dihydro-2(1H)-oxochinazolin-3-yl]-1-piperidinyl]carbonyl]-D-tyrosyl]-4-(1-methyl-4-piperidinyl)-piperazin,

(CG)   1-[4-Amino-3,5-dibrom-N-[[4-[1,3-dihydro-4-(3-nitrophenyl)-2(2H)-oxoimidazol-1-yl]-1-piperidinyl]carbonyl]-D-phenylalanyl]-4-(1-methyl-4-piperidinyl)-piperidin,

(CH)    1-(4-Amino-3,5-dibrom-N-[[4-[3,4-dihydro-2(1H)-oxochinazolin-3-yl]-1-piperidinyl]carbonyl]-D-phenylalanyl]-4-(1-pyrrolidinyl)-piperidin,

(CI)    1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(hexahydro-1H-1-azepinyl)-piperidin und

(CJ)    1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperazin,

deren Tautomere, deren Diastereomere, deren Enantiomere, deren Gemische und deren physiologisch verträglichen Salze, wobei die Verbindungen

(A)  1-[N$^2$-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,

(B)  1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,

(I)  1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-piperidinyl)-piperidin,

(J)  1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazin,

(AC)  (R,S)-1-[4-[4-(3,4-Dihydro-2(1H)-oxochinazolin-3-yl)-1-piperidinyl]-2-[(3,5-dibrom-4-methylphenyl)methyl]-1,4-dioxobutyl]-4-(4-methyl-1-piperazinyl)-piperidin,

(AF) 1-[N$^2$-[N-[[4-(1,3-Dihydro-2(2H)-oxobenzimidazol-1-yl)-1-piperidinyl]carbonyl]-3,5-dibrom-D-tyrosyl]-L-lysyl]-4-(4-pyridinyl)-piperazin und

(AM)  1-[4-Amino-3,5-dibrom-N-[[4-(3,4-dihydro-2(1H)-oxochinazolin-3-yl)-1-piperidinyl]carbonyl]-D-phenylalanyl]-4-(1-piperidinyl)-piperidin,

deren Tautomere, deren Diastereomere, deren Enantiomere, deren Gemische und deren physiologisch verträglichen Salze, insbesondere jedoch die Verbindungen

(A)  1-[N$^2$-(3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin und

(B)  1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,

deren Tautomere, deren Diastereomere, deren Enantiomere, deren Gemische und deren physiologisch verträglichen Salze besonders bevorzugt sind.

Die zur Erzielung einer entsprechenden Wirkung erforderliche Dosierung beträgt bei intravenöser oder subcutaner Gabe zweckmäßig 0,0001 bis 3 mg/kg Körpergewicht, vorzugsweise 0,01 bis 1 mg/kg Körpergewicht, und bei oraler, nasaler oder inhalativer Gabe 0,01 bis 10 mg/kg Körpergewicht, vorzugsweise 0,1 bis 10 mg/kg Körpergewicht, jeweils 1 bis 3 x täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Hierzu lassen sich die CGRP-Antagonisten oder/und CGRP-Release-Hemmer zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen einarbeiten.

Besonders geeignet zur Bekämpfung menopausaler Hitzewallungensind Arzneimittel, enthaltend einen der Wirkstoffe

(A)  1-[N$^2$-[3,  5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,

(B)  2-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,

(I)  1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-piperidinyl)-piperidin,

(J)     1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazin,

(AC)   (R,S)-1-[4-[4-(3,4-Dihydro-2(1H)-oxochinazolin-3-yl)-1-piperidinyl]-2-[(3,5-dibrom-4-methylphenyl)methyl]-1,4-dioxobutyl]-4-(4-methyl-1-piperazinyl)-piperidin,

(AF) 1-[N$^2$-[N-[[4-(1,3-Dihydro-2(2H)-oxobenzimidazol-1-yl)-1-piperidinyl]carbonyl]-3,5-dibrom-D-tyrosyl]-L-lysyl]-4-(4-pyridinyl)-piperazin oder

(AM)    1-[4-Amino-3,5-dibrom-N-[[4-(3,4-dihydro-2(1H)-oxochinazolin-3-yl)-1-piperidinyl]carbonyl]-D-phenylalanyl]-4-(1-piperidinyl)-piperidin,

in einer der folgenden pharmazeutischen Darreichungsformen:

Kapseln zur Pulverinhalation mit 1 mg Wirkstoff, vorzugsweise Wirkstoff (A) oder (B),

Inhalationslösung für Vernebler mit 1 mg Wirkstoff, vorzugsweise Wirkstoff (A) oder (B),

Treibgas-Dosieraerosol mit 1 mg Wirkstoff, vorzugsweise Wirkstoff (A) oder (B),

Nasalspray mit 1 mg Wirkstoff, vorzugsweise Wirkstoff (A) oder (B),

Tabletten mit 20 mg Wirkstoff, vorzugsweise Wirkstoff (B),

Kapseln mit 20 mg Wirkstoff, vorzugsweise Wirkstoff (B),

wäßrige Lösung für die nasale Applikation mit 10 mg Wirkstoff, vorzugsweise Wirkstoff (A) oder (B),

wäßrige Lösung für die nasale Applikation mit 5 mg Wirkstoff, vorzugsweise Wirkstoff (A) oder (B), oder

Suspension für die nasale Applikation mit 20 mg Wirkstoff, vorzugsweise Wirkstoff (A) oder (B).

CGRP wird von sensorischen Nerven freigesetzt, beispielsweise vom Nervus Trigeminus, der einen Teil der Gesichtshaut innerviert. Es wurde bereits gezeigt, daß die Reizung des Trigeminalganglions bei Menschen zu einer Erhöhung des CGRP Plasmaspiegels führt und Gesichtsröte hervorruft ([4]: P.J. Goadsby et al., Annals of Neurology, Vol. 23, No. 2, 1988, 193-196,).
Zum Nachweis, daß mit Hilfe von CGRP-Antagonisten und CGRP-Release-Hemmern Hitzewallungen erfolgreich behandelt werden können, wurde eine erhöhte Freisetzung von endogenem CGRP bei Marmosets durch Stimulierung des Trigeminusganglions herbeigeführt, was zu einer erhöhten Durchblutung der Hautgefäße führte. Die Wirksamkeit folgender Testsubstanzen wurde durch Bestimmung derjenigen i.v.-applizierten Dosis charakterisiert, welche die durch endogenes CGRP hervorgerufene, erhöhte Durchblutung der Gesichtshaut um 50% reduziert:

(A)  =  1-[N$^2$-[3, 5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,

(B)  =  1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,

(AC)   =   (R,S)-1-[4-[4-(3,4-Dihydro-2(1H)-oxochinazolin-3-yl)-1-piperidinyl]-2-[(3,5-dibrom-4-methylphenyl)methyl]-1,4-dioxobutyl]-4-(4-methyl-1-piperazinyl)-piperidin,

(AM)  =  1-[4-Amino-3,5-dibrom-N-[[4-(3,4-dihydro-2(1H)-oxochinazolin-3-yl)-1-piperidinyl]carbonyl]-D-phenylalanyl]-4-(1-piperidinyl)-piperidin,

(DA) = Sumatriptan und

(DB) = Zolmitriptan.

Methodenbeschreibung:

Marmosets beiderlei Geschlechts (300-400 g) werden mit Pentobarbital narkotisiert (Einleitung mit 30 mg/kg, i.p., Infusion 6mg/kg/h, i.m.). Die Körpertemperatur wird über eine Wärmeplatte bei 37 °C gehalten. Als Muskelrelaxants wird Pankurmium (initial 1mg/kg, 0.5 mg nach jeder weiteren Stunde) verabreicht. Der Kopf der Tiere wird in einem stereotaktischen Gerät fixiert. Nachdem die Kopfhaut durch einen Längsschnitt geöffnet wurde, wird ein kleines Loch in den Schädel gebohrt und eine bipolare Elektrode (Rhodes SNES 100) ins Trigeminalganglion abgesenkt.

Die Auffindung des Ganglions wird durch eine Röntgenaufnahme, die die knöcherne Schädelstruktur sichtbar macht, erleichtert. Dabei dient das Felsenbein als Orientierung zur Platzierung der Elektrode (Röntgengerät CCX-Digital). Die Position der Elektrode im Ganglion wird am Ende jedes Experiments kontrolliert. Die Stimulationsparameter sind: 10 Hz, 2 mA, 2 msec, 30 sec lang.

Der Blutfluß in den Mikrogefäßen der Gesichtshaut wird mittels Laser-Doppler-Flußmessung mit einem PeriFlux Laser Doppler System erfaßt.

Die Tiere werden 2 bis 3 Stimulationsperioden im Abstand von jeweils 30 min ausgesetzt. Dabei dient die erste Stimulation als Bezugswert für die weiteren Stimulationen. Die Testsubstanzen werden i.v. 5 min vor der 2. bzw. 3. Stimulationsperiode appliziert.

Tabelle 1:

| "50%-Dosis" = i.v. Dosis, welche die durch endogenes CGRP hervorgerufene, erhöhte Durchblutung der Gesichtshaut um 50% reduziert ||
|---|---|
| Substanz | 50%-Dosis |
| A | 0.003 mg/kg |
| B | 0.042 mg/kg |
| AC | 0.018 mg/kg |
| AM | 0.046 mg/kg |
| DA | 0.280. mg/kg |
| DB | 0.035 mg/kg |

Die nachfolgenden Beispiele beschreiben pharmazeutische Anwendungsformen, die als Wirkstoff einen CGRP-Antagonisten oder CGRP-Release-Hemmer für die erfindungsgemäße Verwendung enthalten, vorzugsweise eines der in der WO 98/11128 oder DE 199 11 039 beschriebenen Aminosäurederivate, beispielsweise einen der vorstehend genannten Wirkstoffe (A) oder (B):

Beispiel I

Kapseln zur Pulverinhalation mit 1 mg Wirkstoff (A) oder (B)

Zusammensetzung:

1 Kapsel zur Pulverinhalation enthält:

| | |
|---|---|
| Wirkstoff (A) oder (B) | 1.0 mg |
| Milchzucker | 20.0 mg |
| Hartgelatinekapseln | 50.0 mg |
| | 71.0 mg |

Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

Beispiel II

Inhalationslösung für Respimat® mit 1 mg Wirkstoff (A) oder (B)

Zusammensetzung:

1 Hub enthält:

| Wirkstoff (A) oder (B) | 1.0 mg |
|---|---|
| Benzalkoniumchlorid | 0.002 mg |
| Dinatriumedetat | 0.0075 mg |
| Wasser gereinigt ad | 15.0 µl |

Herstellungsverfahren:

Der Wirkstoff und Benzalkoniumchlorid werden in Wasser gelöst und in Respimat®-Kartuschen abgefüllt.

Beispiel III

Inhalationslösung für Vernebler mit 1 mg Wirkstoff (A) oder (B)

Zusammensetzung:

1 Fläschchen enthält:

| Wirkstoff (A) oder (B) | 0.1 g |
|---|---|
| Natriumchlorid | 0.18 g |
| Benzalkoniumchlorid | 0.002 g |
| Wasser gereinigt ad | 20.0 ml |

Herstellungsverfahren:

Wirkstoff, Natriumchlorid und Benzalkoniumchlorid werden in Wasser gelöst.

Biespiel IV

Treibgas-Dosieraerosol mit 1 mg Wirkstoff (A) oder (B)

Zusammensetzung:

1 Hub enthält:

| Wirkstoff (A) oder (B) | 1.0 mg |
|---|---|
| Lecithin | 0.1 % |
| Treibgas ad | 50.0 µl |

Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

Beispeil V

Nasalspray mit 1 mg Wirkstoff (A) oder (B)

Zusammensetzung:

1 Sprühstoss enthält

| Wirkstoff (A) oder (B) | 1.0 mg |
|---|---|
| Mannit | 5.0 mg |
| Dinatriumedetat | 0.05 mg |
| Ascorbinsäure | 1.0 mg |
| Wasser gereinigt ad | 0.1 ml |

Herstellungsverfahren:

Der Wirkstoff und die Hilfsstoffe werden in Wasser gelöst und in ein entsprechendes Behältnis abgefüllt.

Beispiel VI

Injektionslösung mit 5 mg Wirksubstanz (A) oder (B) pro 5 ml

Zusammensetzung:

| Wirksubstanz (A) oder (B) in basischer Form | 5 mg |
|---|---|
| Säure/Salzbildner in der zur Bildung eines neutralen Salzes erforderlichen Menge | q.s. |
| Glucose | 250 mg |
| Human-Serum-Albumin | 10 mg |
| Glykofurol | 250 mg |
| Wasser für Injektionszwecke ad | 5 ml |

Herstellung:

Glykofurol und Glucose in Wasser für Injektionszwecke auflösen (WfI); Human-Serum-Albumin zugeben; Salzbildner zugeben; Wirkstoff unter Erwärmen auflösen; mit WfI auf Ansatzvolumen auffüllen; unter Stickstoff-Begasung in Ampullen abfüllen.

Beispiel VII

Injektionslösung für die subcutane Applikation mit 5 mg Wirksubstanz (A) oder (B) pro 1 ml

Zusammensetzung:

| Wirksubstanz (A) oder (B) | 5 mg |
|---|---|
| Glucose | 50 mg |
| Polysorbat 80 = Tween 80 | 2 mg |
| Wasser für Injektionszwecke ad | 1 ml |

Herstellung:

Glucose und Polysorbat in Wasser für Injektionszwecke auflösen; Wirkstoff unter Erwärmen bzw. mittels Ultraschall auflösen; mit WfI auf Ansatzvolumen auffüllen; unter Inertgasbegasung in Ampullen abfüllen.

Beispiel VIII

Injektionslösung mit 100 mg Wirksubstanz (A) oder (B) pro 10 ml

Zusammensetzung:

| | |
|---|---|
| Wirksubstanz (A) oder (B) | 100 mg |
| Monokaliumdihydrogenphosphat = $KH_2PO_4$ | 12 mg |
| Dinatriumhydrogenphosphat = $Na_2HPO_4 \cdot 2H_2O$ | 2 mg |
| Natriumchlorid | 180 mg |
| Human-Serum-Albumin | 50 mg |
| Polysorbat 80 | 20 mg |
| Wasser für Injektionszwecke ad | 10 ml |

Herstellung:

Polysorbat 80, Natriumchlorid, Monokaliumdihydrogenphosphat und Dinatriumhydrogenphosphat in Wasser für Injektionszwecke (WfI) auflösen; Human-Serum-Albumin zugeben; Wirkstoff unter Erwärmen auflösen; mit WfI auf Ansatzvolumen auffüllen; in Ampullen abfüllen.

Beispiel IX

Lyophilisat mit 10 mg Wirksubstanz (A) oder (B)

Zusammensetzung:

| | |
|---|---|
| Wirksubstanz (A) oder (B) in basischer Form | 10 mg |
| Säure/Salzbildner in der zur Bildung eines neutralen Salzes erforderlichen Menge | q.s. |
| Mannit | 300 mg |
| Wasser für Injektionszwecke ad | 2 ml |

Herstellung :

Mannit in Wasser für Injektionszwecke (WfI) auflösen; Salzbildner zugeben; Wirkstoff unter Erwärmen auflösen; mit WfI auf Ansatzvolumen auffüllen; in Vials abfüllen; gefriertrocknen.

Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

Herstellung:

Polysorbat 80 und Mannit in Wasser für Injektionszwecke (WfI) auflösen; in Ampullen abfüllen.

Beispiel X

Lyophilisat mit 5 mg Wirksubstanz (A) oder (B)

Zusammenstzung:

| Wirkstoff (A) oder (B) in basischer Form | 5 mg |
| --- | --- |
| polares oder unpolares Lösungsmittel (welches mittels Gefriertrocknung entfernt werden kann) ad | 1 ml |

Herstellung:

Wirkstoff in geeignetem Lösungsmittel auflösen; in Vials abfüllen; gefriertrocknen.

Lösungsmittel für Lyophilisat :

| Polysorbat 80 = Tween 80 | 5 mg |
| --- | --- |
| Mannit | 100 mg |
| Wasser für Injektionszwecke ad | 2 ml |

Herstellung:

Polysorbat 80 und Mannit in Wasser für Injektionszwecke (WfI) auflösen; in Ampullen abfüllen.

Beispiel XI

Tabletten mit 20 mg Wirksubstanz (A) oder (B)

Zusammensetzung:

| Wirksubstanz (A) oder (B) | 20 mg |
| --- | --- |
| Lactose | 120 mg |
| Maisstärke | 40 mg |
| Magnesiumstearat | 2 mg |
| Povidon K 25 | 18 mg |

Herstellung :

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

Beispiel XII

Kapseln mit 20 mg Wirksubstanz (A) oder (B)

Zusammensetzung:

| Wirksubstanz (A) oder (B) | 20 mg |
| --- | --- |

(fortgesetzt)

| Maisstärke | 80 mg |
|---|---|
| Kieselsäure, hochdispers | 5 mg |
| Magnesiumstearat | 2.5 mg |

Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

Beispiel XIII

Zäpfchen mit 50 mg Wirksubstanz (A) oder (B)

Zusammensetzung:

| Wirksubstanz (A) oder (B) | 50 mg |
|---|---|
| Hartfett (Adeps solidus) q.s. ad | 1700 mg |

Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

Beispeil XIV

Wäßrige Lösung für die nasale Applikation mit 10 mg Wirksubstanz (A) oder (B)

Zusammensetzung:

| Wirksubstanz (A) oder (B) | 10.0 mg |
|---|---|
| Salzsäure in der zur Bildung eines neutralen Salzes erforderlichen Menge | |
| Parahydroxybenzoesäuremethylester (PHB) | 0.01 mg |
| Parahydroxybenzoesäurepropylester (PHB) | 0.005 mg |
| Wasser gereinigt ad | 1.0 ml |

Herstellung:

Der Wirkstoff wird in gereinigtem Wasser aufgelöst; Salzsäure wird zugegeben, bis die Lösung klar wird; PHB-Methyl- und Propylester werden zugegeben; die Lösung wird mit gereinigtem Wasser auf Ansatzvolumen aufgefüllt; die Lösung wird sterilfiltriert und in ein entsprechendes Behältnis abgefüllt.

Beispiel XV

Wäßrige Lösung für die nasale Applikation mit 5 mg Wirksubstanz (A) oder (B)

Zusammensetzung:

| Wirksubstanz (A) oder (B) | 5 mg |
|---|---|

(fortgesetzt)

| 1,2-Propandiol | 300 mg |
|---|---|
| Hydroxyethylcellulose | 5 mg |
| Sorbinsäure | 1 mg |
| Wasser gereinigt ad | 1 ml |

Herstellung:

Der Wirkstoff wird in 1,2-Propandiol gelöst; eine Hydroxyethyl-cellulose-Lösung in gereinigtem Wasser enthaltend Sorbinsäure wird hergestellt und zur Wirkstoff-Lösung gegeben; die Lösung wird sterilfiltriert und in ein entsprechendes Behältnis abgefüllt.

Beispiel XVI

Wäßrige Lösung für die intravenöse Applikation mit 5 mg Wirksubstanz (A) oder (B)

Zusammensetzung:

| Wirksubstanz (A) oder (B) | 5 mg |
|---|---|
| 1,2-Propandiol | 300 mg |
| Mannit | 50 mg |
| Wasser für Injektionszwecke (WfI) ad | 1 ml |

Herstellung:

Der Wirkstoff wird in 1,2-Propandiol gelöst; die Lösung wird mit WfI auf annähernd Ansatzvolumen aufgefüllt; das Mannit wird zugegeben und mit WfI auf Ansatzvolumen aufgefüllt; die Lösung wird sterilfiltriert, in Einzelbehältnisse abgefüllt und autoklaviert.

Beispiel XVII

Liposomale Formulierung für die intravenöse Injektion mit 7.5 mg Wirksubstanz (A) oder (B)

Zusammensetzung:

| Wirksubstanz (A) oder (B) | 7.5 mg |
|---|---|
| Ei-lecithin, z.B. Lipoid E 80 | 100.0 mg |
| Cholesterol | 50.0 mg |
| Glycerin | 50.0 mg |
| Wasser für Injektionszwecke ad | 1.0 ml |

Herstellung:

Der Wirkstoff wird in einer Mischung aus Lecithin und Cholesterol gelöst; die Lösung wird zu einer Mischung aus Glycerin und WfI gegeben und mittels Hochdruck-Homogenisation oder Microfluidizer-Technik homogenisiert; die so erhaltene liposomale Formulierung wird unter aseptischen Bedingungen in ein entsprechendes Behältnis abgefüllt.

Beispiel XVIII

Suspension für die nasale Applikation mit 20 mg Wirksubstanz (A) oder (B)

Zusammensetzung:

| Wirksubstanz (A) oder (B) | 20.0 mg |
|---|---|
| Carboxymethylcellulose (CMC) | 20.0 mg |
| Natriummonohydrogenphosphat/Natriumdihydrogenphosphat-Puffer pH 6.8 | q.s. |
| Natriumchlorid | 8.0 mg |
| Parahydroxybenzoesäuremethylester | 0.01 mg |
| Parahydroxybenzoesäurepropylester | 0.003 mg |
| Wasser gereinigt ad | 1.0 ml |

Herstellung :

Der Wirkstoff wird in einer wässrigen CMC-Lösung suspendiert; die anderen Bestandteile werden nacheinander zur Suspension gegeben und die Suspension mit gereinigtem Wasser auf Ansatzvolumen aufgefüllt.

Beispiel XIX

Wässrige Lösung für die subcutane Applikation mit 10 mg Wirksubstanz (A) oder (B)

Zusammensetzung:

| Wirksubstanz (A) oder (B) | 10.0 mg |
|---|---|
| Natriummonohydrogenphosphat/Natriumdihydrogenphosphat-Puffer q.s. ad pH | 7.0 |
| Natriumchlorid | 4.0 mg |
| Wasser für Injektionszwecke ad | 0.5 ml |

Herstellung:

Der Wirkstoff wird in der Phosphatpufferlösung gelöst, nach Zugabe des Kochsalz wird mit Wasser auf Ansatzvolumen aufgefüllt. Die Lösung wird sterilfiltriert und nach Abfüllung in ein entsprechendes Behältnis autoklaviert.

Biespiel XX

Wässrige Suspension für die subcutane Applikation mit 5 mg Wirksubstanz (A) oder (B)

Zusammensetzung:

| Wirksubstanz (A) oder (B) | 5.0 mg |
|---|---|
| Polysorbat 80 | 0.5 mg |
| Wasser für Injektionszwecke | 0.5 ml |

Herstellung:

Der Wirkstoff wird in der Polysorbat 80-Lösung suspendiert und mittels geeigneter Dispiergiertechnik (z.B. Naßmahlung, Hochdruckhomogenisation, Mikrofluidisierung etc.) auf eine Teilchengröße von ca. 1 μm zerkleinert. Die Suspension wird unter aseptischen Bedingungen in ein entsprechendes Behältnis abgefüllt.

**Patentansprüche**

1.  Verwendung eines CGRP-Antagonisten zur Herstellung eines Arzneimittels zur Bekämpfung menopausaler Hitzewallungen , **dadurch gekennzeichnet, dass** der CGRP-Antagonist ausgewählt ist aus der Gruppe bestehend aus:

    (A) 1-[N$^2$-[3,5-Dibrom-N-[[4-(3,4-dihydro-2(1H)-oxochinazolin-3-yl)-1-piperidinyl]carbonyl]-D-tyrosyl]-L-lysyl]-4-(4-pyridinyl)-piperazin,

    (B)   1-[4-Amino-3,5-dibrom-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidin,

    (C)   1-[N$^2$-[4-Amino-3,5-dibrom-N-[[4-(3,4-dihydro-2(1H)-oxochinazolin-3-yl)-1-piperidinyl]carbonyl]-D-phenylalanyl]-L-lysyl]-4-(4-pyridinyl)-piperazin,

    (D)   1-[N$^2$-[4-Amino-3,5-dibrom-N-[[4-(3,4-dihydro-2(1H)-oxochinazolin-3-yl)-1-piperidinyl]carbonyl]-D-phenylalanyl]-L-lysyl]-4-(4-pyridinyl)-piperidin,

    (E)   1-[N$^2$-[3,5-Dibrom-N-[[4-(1,3-dihydro-4-phenyl-2(2H)-oxoimidazol-1-yl)-1-piperidinyl]carbonyl]-D-tyrosyl]-L-lysyl]-4-(4-pyridinyl)-piperazin,

    (F)   1-[N$^2$-[4-Amino-3,5-dibrom-N-[[4-(1,3-dihydro-4-phenyl-2(2H)-oxoimidazol-1-yl)-1-piperidinyl]carbonyl]-D-phenylalanyl]-L-lysyl]-4-(4-pyridinyl)-piperazin,

    (G)   1-[3,5-Dibrom-N-[[4-(3,4-dihydro-2(1H)-oxothieno[3,4-d]-pyrimidin-3-yl)-1-piperidinyl]carbonyl]-D-tyrosyl]-4-(1-piperidinyl)-piperidin,

    (H)   1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperidin,

    (I) 1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-piperidinyl)-piperidin,

    (J)   1-[4-Amino-3,5-dibrom-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazin,

    (K)   1-[4-Amino-3,5-dibrom-N-[[4-(3,4-dihydro-2(1H)-oxothieno[3,2-d]pyrimidin-3-yl)-1-piperidinyl]carbonyl]-D-phenylalanyl]-4-(1-piperidinyl)-piperidin,

    (L) 1-[4-Amino-3,5-dibrom-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-ethyl-4-piperidinyl)-piperidin,

    (M) 1-[4-Amino-3,5-dibrom-N-[[4-[3,4-dihydro-2(1H)-oxochinazolin-3-yl]-1-piperidinyl]carbonyl]-D-phenylalanyl]-4-(1-hexyl-4-piperidinyl)-piperidin,

    (N) 1-[4-Amino-3,5-dibrom-N-[[4-[3,4-dihydro-2(1H)-oxochinazolin-3-yl]-1-piperidinyl]carbonyl]-D-phenylalanyl]-4-(1-cyclopropylmethyl-4-piperidinyl)-piperidin,

    (O)1-[N-[[4-[3,4-Dihydro-2(1H)-oxochinazolin-3-yl]-1-piperidinyl]carbonyl]-3-ethenyl-D,L-phenylalanyl]-4-(hexahydro-1H-1-azepinyl)-piperidin,

    (P) (R,S)-1-[4-[4-(3,4-Dihydro-2(1H)-oxochinazolin-3-yl)-1-piperidinyl]-2-[(4-hydroxy-3,5-dimethylphenyl)methyl]-1,4-dioxobutyl]-4-(1-piperidinyl)-piperidin,

    (Q)   1-[4-Amino-3,5-dibrom-N-[[4-[N-(aminocarbonyl)-N-phenylamino]-1-piperidinyl]carbonyl]-D-phenylalanyl]-4-(1-piperidinyl)-piperidin,

    (R) 1-[4-Amino-3,5-dibrom-N-[[4-(3,4-dihydro-2(1H)-oxochinazolin-3-yl)-1-piperidinyl]carbonyl]-D-phenylalanyl]-4-(5-methoxy-4-pyrimidinyl)-piperazin,

(S) 1-[4-Amino-3,5-dibrom-N-[[4-(1,1-dioxido-3(4H)-oxo-1,2,4-benzothiadiazin-2-yl)-1-piperidinyl]carbonyl]-D-phenylalanyl]-4-(1-piperidinyl)-piperidin,

(T)1-[4-Amino-3,5-dibrom-N-[[4-[2(1H)-oxochinolin-3-yl]-1-piperidinyl]carbonyl]-D-phenylalanyl]-4-(1-piperidinyl)-piperidin,

(U) 1-[4-Amino-3,5-dibrom-N-[[4-[3,4-dihydro-2(1H)-oxochinazolin-3-yl]-1-piperidinyl]carbonyl]-D-phenylalanyl]-4-[3-(dimethylamino)propyl]-piperazin,

(V) 1-[4-Amino-3,5-dibrom-N-[[4-[3,4-dihydro-2(1H)-oxochinazolin-3-yl]-1-piperidinyl]carbonyl]-D-phenylalanyl]-4-(4-methyl-1-piperazinyl)-piperidin,

(W) 1-[4-Amino-3,5-dibrom-N-[[4-[3,4-dihydro-2(1H)-oxochinazolin-3-yl]-1-piperidinyl]carbonyl]-D-phenylalanyl]-4-[(1-methyl-4-piperidinyl)carbonyl]-piperazin,

(X) 1-[4-Amino-3,5-dibrom-N-((4-[3,4-dihydro-2(1H)-oxochinazolin-3-yl]-1-piperidinyl]carbonyl]-D-phenylalanyl]-4-[(1-methyl-4-piperazinyl)carbonyl]-piperazin,

(Y) 1-[4-Amino-3,5-dibrom-N-[[4-(3,4-dihydro-2(1H)-oxochinazolin-3-yl)-1-piperidinyl]carbonyl]-D-phenylalanyl]-4-[4-[4-(dimethylamino)butyl]phenyl]-piperazin,

(Z) 1-[4-Amino-3,5-dibrom-N-[(4-(3,4-dihydro-2(1H)-oxochinazolin-3-yl)-1-piperidinyl]carbonyl]-D-phenylalanyl]-4-[4-(dimethylamino)-1-piperidinyl]-piperidin,

(AA) 1-[$N^2$-[[4-(1,3-Dihydro-4-phenyl-2(2H)-oxoimidazol-1-yl)-1-piperidinyl]carbonyl-N'-methyl-D-tryptyl]-4-(4-methyl-1-piperazinyl)-piperidin,

(AB) 2-[$N^2$-[[4-(1,3-Dihydro-4-phenyl-2(2H)-oxoimidazol-1-yl)-1-piperidinyl]carbonyl]-N'-(1,1-dimethylethoxycarbonyl)-D-tryptyl]-4-(1-methyl-4-piperidinyl)-piperidin,

(AC) (R,S)-1-[4-[4-(3,4-Dihydro-2(1H)-oxochinazolin-3-yl)-1-piperidinyl]-2-[(3,5-dibrom-4-methylphenyl)methyl]-1,4-dioxobutyl]4-(4-methyl-1-piperazinyl)-piperidin,

(AD) (R,S)-1-[4-[4-(3,4-Dihydro-2(1H)-oxochinazolin-3-yl)-1-piperidinyl]-2-[(3,5-dibrom-4-methoxyphenyl)methyl]-1,4-dioxobutyl]-4-(1-methyl-4-piperidinyl)-piperidin,

(AE) (R,S)-1-[4-[4-(3,4-Dihydro-2(1H)-oxochinazolin-3-yl)-1-piperidinyl]-2-[(3,4-dibromphenyl)methyl]-1,4-dioxobutyl]-4-(4-methyl-1-piperazinyl)-piperidin,

(AF) 1-[$N^2$-[N-[[4-(1,3-Dihydro-2(2H)-oxobenzimidazol-1-yl)-1-piperidinyl]carbonyl]-3,5-dibrom-D-tyrosyl]-L-lysyl]-4-(4-pyridinyl)-piperazin,

(AG) 1-[4-Amino-3,5-dibrom-N-[[4-(1,3-dihydro-6-hydroxy-2(2H)-oxobenzimidazol-1-yl)-1-piperidinyl]carbonyl]-D-phenylalanyl]-4-(1-piperidinyl)-piperidin,

(AH) 1-[$N^2$-[4-Amino-3,5-dibrom-N-[[4-(1,3-dihydro-2(2H)-oxobenzimidazol-1-yl)-1-piperidinyl]carbonyl]-D-phenylalanyl]-$N^6$,$N^6$-dimethyl-L-lysyl]-4-(4-pyridinyl)-piperazin,

(AI) 1-[$N^2$-[4-Amino-3,5-dibrom-N-[[4-(3,4-dihydro-2(1H)-oxochinazolin-3-yl)-1-piperidinyl]carbonyl]-D-phenylalanyl]-$N^6$,$N^6$-dimethyl-L-lysyl]-4-(4-pyridinyl)-piperazin,

(AJ) (R,S)-1-[2-(4-Amino-3,5-dibrombenzoyl)-4-[4-(3,4-dihydro-2(1H)-oxochinazolin-3-yl)-1-piperidinyl]-4-oxobutyl]-4-(1-piperidinyl)-piperidin,

(AK) 1-[4-Amino-3,5-dibrom-N-[[4-(3,4-dihydro-2,2-dioxido-2,1,3-benzothiadiazin-3-yl)-1-piperidinyl]carbonyl]-D-phenylalanyl]-4-(1-piperidinyl)-piperidin,

(AL)  1-[4-Amino-3,5-dibrom-N-[[4-[1,3-dihydro-2(2H)-oxoimidazo[4,5-c]chinolin-3-yl]-1-piperidinyl]carbonyl]-D-phenylalanyl]-4-(1-piperidinyl)carbonyl)-piperidin,

(AM)  1-[4-Amino-3,5-dibrom-N-[[4-(3,4-dihydro-2(1H)-oxochinazolin-3-yl)-1-piperidinyl]carbonyl]-D-phenyl-alanyl]-4-(1-piperidinyl)-piperidin,

(AN)  1-[N$^2$-[3,5-Dibrom-N-[[4-(3,4-dihydro-2(1H)-oxochinazolin-3-yl)-1-piperidinyl]carbonyl]-D-tyrosyl]-N$^6$, N$^6$-dimethyl-L-lysyl]-4-(4-pyridinyl)-piperazin,

(AO)  1-[4-Amino-N-[[4-[4-(3-bromphenyl)-1,3-dihydro-2(2H)-oxoimidazol-1-yl]-2-piperidinyl]carbonyl]-3,5-dibrom-D-phenylalanyl]-4-(1-methyl-4-piperidinyl)-piperidin,

(AP)  2-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(4-methyl-1-piperazinyl)-piperidin,

(AQ)  1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-piperidinyl)-piperidin,

(AR)  1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(exo-8-methyl-8-azabicyclo[3,2,1]oct-3-yl)-piperazin,

(AS)  1-[3,5-Dibrom-N-[[4-(1,3-dihydro-4-phenyl-2(2H)-oxoimidazol-1-yl)-1-piperidinyl]carbonyl]-D-tyrosyl]-4-(2-piperidinyl)-piperidin,

(AT) 1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-ethyl-4-piperidinyl)-piperazin,

(AU)  1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-(hexahydro-4-methyl-1H-1,4-diazepin-1-yl)piperidin,

(AV)  1-[3,5-Dibrom-N-[[4-(3,4-dihydro-2(1H)-oxochinazolin-3-yl)-1-piperidinyl]carbonyl]-D-tyrosyl]-4-[1-(methylsulfonyl)-4-piperidinyl]-piperidin,

(AW)  1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperidin,

(AX)  1-[3,5-Dibrom-N-[[4-[1,3-dihydro-4-[3-(trifluormethyl)-phenyl]-2(2H)-oxoimidazol-1-yl]-1-piperidinyl]carbonyl]-D-tyrosyl]-4-(hexahydro-1H-1-azepinyl)-piperidin,

(AY)  1-[3,5-Dibrom-N-[[4-(3,4-dihydro-2(1H)-oxochinazolin-3-yl)-1-piperidinyl]carbonyl]-D-tyrosyl]-4-(1-methyl-4-piperidinyl)-piperidin,

(AZ)  1-[4-Amino-3,5-dibrom-N-[[4-(3,4-dihydro-2(1H)-oxochinazolin-3-yl)-1-piperidinyl]carbonyl]-D-phenyl-alanyl]-4-(exo-8-methyl-8-azabicyclo[3,2,1]oct-3-yl)-piperazin,

(BA)  1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperazin,

(BB)  1-[3,5-Dibrom-N-[[4-[1,3-dihydro-4-[3-(trifluormethyl)-phenyl]-2(2H)-oxoimidazol-1-yl]-1-piperidinyl]carbonyl]-D-tyrosyl]-4-(1-piperidinyl)-piperidin,

(BC) 1-[N$^6$-Acetyl-N$^2$-[3,5-dibrom-N-[[4-(3,4-dihydro-2(1H)-oxochinazolin-3-yl)-1-piperidinyl]carbonyl]-D-tyrosyl]-Llysyl]-4-(4-pyridinyl)-piperazin,

(BD)  1-[3,5-Dibrom-N-[[4-(1,3-dihydro-4-phenyl-2(2H)-oxoimidazol-1-yl)-1-piperidinyl]carbonyl]-D-tyrosyl]-4-(hexahydro-1H-1-azepinyl)-piperidin,

(BE)  1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperidin,

(BF)   1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-methyl-4-piperidinyl)-piperidin,

(BG)-[3,5-Dibrom-N-[[4-(3,4-dihydro-2(1H)-oxochinazolin-3-yl)-1-piperidinyl]carbonyl]-D-tyrosyl]-4-[1-(hydroxycarbonylmethyl)-4-piperidinyl]-piperidin,

(BH)   1-[4-Amino-3,5-dibrom-N-[[4-[3,4-dihydro-2(1H)-oxochinazolin-3-yl]-1-piperidinyl]carbonyl]-D-phenylalanyl]-4-(1-methylsulfonyl-4-piperidinyl)-piperidin,

(BI)  1-[3,5-Dibrom-N-[[4-(3,4-dihydro-2(1H)-oxochinazolin-3-yl)-1-piperidinyl]carbonyl]-D-tyrosyl]-4-(4-piperidinyl)-piperidin,

(BJ)   1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-ethyl-4-piperidinyl)-piperidin,

(BK)   1-[4-Amino-3,5-dibrom-N-[[4-(1,3-dihydro-4-(3-hydroxyphenyl)-2(2H)-oxoimidazol-1-yl)-1-piperidinyl]carbonyl]-D-phenylalanyl]-4-(1-methyl-4-piperidinyl)-piperidin,

(BL) 1-[3,5-Dibrom-N-[[4-(3,4-dihydro-2(1H)-oxochinazolin-3-yl)-1-piperidinyl]carbonyl]-D-tyrosyl]-4-(hexahydro-1H-1-azepinyl)-piperidin,

(BM)   1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(1-piperidinyl)-piperidin,

(BN)   1-[4-Amino-3,5-dibrom-N-[[4-[4-(3-bromphenyl)-1,3-dihydro-2(2H)-oxoimidazol-1-yl]-1-piperidinyl]carbonyl]-D-phenylalanyl]-4-(exo-8-methyl-8-azabicyclo[3,2,1]oct-3-yl)-piperazin,

(BO)   1-[4-Amino-3,5-Dibrom-N-[[4-(3,4-dihydro-2(1H)-oxochinazolin-3-yl)-1-piperidinyl]carbonyl]-D-phenylalanyl]-4-(1-ethyl-4-piperidinyl)-piperidin,

(BP)   1-[4-Amino-3,5-Dibrom-N-[[4-(3,4-dihydro-2(1H)-oxochinazolin-3-yl)-1-piperidinyl]carbonyl]-D-phenylalanyl]-4-(1-ethyl-4-piperidinyl)-piperazin,

(BQ)   1-[4-Amino-3,5-dibrom-N-[[4-[1,3-dihydro-4-(3-methoxyphenyl)-2(2H)-oxoimidazol-1-yl]-1-piperidinyl]carbonyl]-D-phenylalanyl]-4-(exo-8-methyl-8-azabicyclo[3,2,1]oct-3-yl)-piperazin,

(BR)1-[3,5-Dibrom-N-[[4-(3,4-dihydro-2(1H)-oxochinazolin-3-yl)-1-piperidinyl]carbonyl]-D-tyrosyl]-4-[1-(cyclopropylmethyl)-4-piperidinyl]-piperidin,

(BS)   1-[4-Amino-3,5-Dibrom-N-[[4-(3,4-dihydro-2(1H)-oxochinazolin-3-yl)-1-piperidinyl]carbonyl]-D-phenylalanyl]-4-(hexahydro-1H-1-azepinyl)-piperidin,

(BT)   1-[4-Amino-3,5-dibrom-N-[[4-(3,4-dihydro-2(1H)-oxochinazolin-3-yl)-1-piperidinyl]carbonyl]-D-phenylalanyl]-4-(4-piperidinyl)-piperidin,

(BU)   1-[3,5-Dibrom-N-[[4-(1,3-dihydro-4-phenyl-2(2H)-oxoimidazol-1-yl)-1-piperidinyl]carbonyl]-D-tyrosyl]-4-(4-pyridinyl)-piperidin,

(BV)   1-[3,5-Dibrom-N-[[4[1,3-dihydro-4-[3-(trifluormethyl)-phenyl]-2(2H)-oxoimidazol-1-yl]-1-piperidinyl]carbonyl]-D-tyrosyl]-4-(1-methyl-4-piperidinyl)-piperazin,

(BW)  2-[N2-[3,5-Dibrom-N-[[4-[1,3-dihydro-4-[3-(trifluormethyl)phenyl]-2(2H)-oxoimidazol-1-yl]-1-piperidinyl]carbonyl]-D-tyrosyl]-L-lysyl]-4-(4-pyridinyl)-piperazin,

(BX)   1-[3,5-Dibrom-N-[[4-(1,3-dihydro-4-(3-thienyl)-2(2H)-oxoimidazol-1-yl)-1-piperidinyl]carbonyl]-D-tyrosyl]-4-(1-piperidinyl)-piperidin,

(BY)   2-[4-Amino-N-[[4-[4-(3-chlorphenyl)-1,3-dihydro-2(2H)-oxoimidazol-1-yl]-1-piperidinyl]carbonyl]-3,5-dibrom-D-phenylalanyl]-4-(2-methyl-4-piperidinyl)-piperidin,

(BZ)   1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(hexahydro-1H-1-azepinyl)-piperidin,

(CA)   1-[4-Amino-3,5-dibrom-N-[[4-[1,3-dihydro-4-[3-(trifluormethyl)phenyl]-2(2H)-oxoimidazol-1-yl]-1-piperidinyl]carbonyl]-D-phenylalanyl]-4-(1-methyl-4-piperidinyl)-piperazin,

(CB)   1-[4-Amino-N-[[4-[4-(3-chlorphenyl)-1,3-dihydro-2(2H)-oxoimidazol-1-yl]-1-piperidinyl]carbonyl]-3,5-dibrom-D-phenylalanyl]-4-(hexahydro-1H-1-azepinyl)-piperidin,

(CC)   1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(4-pyridinyl)-piperazin,

(CD)   1-[3,5-Dibrom-N-[[4-(1,3-dihydro-4-phenyl-2(2H)-oxoimidazol-1-yl)-1-piperidinyl]carbonyl]-D-tyrosyl]-4-(1-methyl-4-piperidinyl)-piperidin,

(CE)   1-[4-Amino-3,5-dibrom-N-[[4-[1,3-dihydro-4-phenyl-2(2H)-oxoimidazol-1-yl]-1-piperidinyl]carbonyl]-D-phenylalanyl]-4-[4-(1-oxoethyl)phenyl]-piperazin,

(CF)   1-[3,5-Dibrom-N-[[4-3-4-dihydro-2(1H)-oxochinazolin-3-yl]-1-piperidinyl]carbonyl]-D-tyrosyl]-4-(1-methyl-4-piperidinyl)-piperazin,

(CG)   1-[4-Amino-3,5-dibrom-N-[[4-[1,3-dihydro-4-(3-nitrophenyl)-2(2H)-oxoimidazol-1-yl]-1-piperidinyl]carbonyl]-D-phenylalanyl]-4-(1-methyl-4-piperidinyl)-piperidin,

(CH)   1-[4-Amino-3,5-dibrom-N-[[4-[3,4-dihydro-2(1H)-oxochinazolin-3-yl]-1-piperidinyl]carbonyl]-D-phenylalanyl]-4-(1-pyrrolidinyl)-piperidin,

(CI)   1-[4-Amino-3,5-dibrom-N-[[4-(1,3-dihydro-4-phenyl-2(2H)-oxoimidazol-1-yl)-1-piperidinyl]carbonyl]-D-phenylalanyl]-4-(hexahydro-1H-1-azepinyl)-piperidin und

(CJ)   1-[4-Amino-3,5-dibrom-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperazin,

deren Tautomere, deren Diastereomere, deren Enantiomere, deren Gemische und deren physiologisch verträglichen Salze.

**2.** Verwendung gemäß Anspruch 1, **dadurch gekennzeichnet, dass** diese in Ergänzung zu einer Hormonsubstitutionstherapie erfolgt.

**3.** Verwendung gemäß Anspruch 1, **dadurch gekennzeichnet, dass** das Arzneimittel nur einen Wirkstoff enthält.

**Claims**

**1.** Use of a CGRP antagonist for preparing a pharmaceutical composition for treating menopausal hot flushes, **characterised in that** the CGRP antagonist is selected from among:

(A)   1-[N$^2$-[3,5-dibromo-N-[[4-(3,4-dihydro-2(1H)-oxoquinazolin-3-yl)-1-piperidinyl]carbonyl]-D-tyrosyl]-L-lysyl]-4-(4-pyridinyl)-piperazine,

(B)   1-[4-amino-3,5-dibromo-N-[[4-(2,3,4,5-tetrahydro-2(1H)-oxo-1,3-benzodiazepin-3-yl)-1-piperidinyl]carbonyl]-D-phenylalanyl]-4-(1-piperidinyl)-piperidine,

(C)   1-[N$^2$-[4-amino-3,5-dibromo-N-[[4-(3,4-dihydro-2(1H)-oxoquinazolin-3-yl)-1-piperidinyl]carbonyl]-D-phenylalanyl]-L-lysyl]-4-(4-pyridinyl)-piperazine,

(D) 1-[N$^2$-[4-amino-3, 5-dibromo-N-[[4-(3, 4-dihydro-2(1H)-oxoquinazolin-3-yl)-1-piperidinyl]carbonyl]-D-phenylalanyl]-L-lysyl]-4-(4-pyridinyl)-piperidine,

(E) 1-[N$^2$-[3,5-dibromo-N-[[4-(1,3-dihydro-4-phenyl-2(2H)-oxoimidazol-1-yl)-1-piperidinyl]carbonyl]-D-tyrosyl]-L-lysyl]-4-(4-pyridinyl)-piperazine,

(F)    1-[N$^2$-[4-amino-3,5-dibromo-N-[[4-(1,3-dihydro-4-phenyl-2(2H)-oxoimidazol-1-yl)-1-piperidinyl]carbonyl]-D-phenylalanyl]-L-lysyl]-4-(4-pyridinyl)-piperazine,

(G)     1-[3,5-dibromo-N-[[4-(3,4-dihydro-2(1H)-oxothieno[3,4-d]pyrimidin-3-yl)-1-piperidinyl]carbonyl]-D-tyrosyl]-4-(1-piperidinyl)-piperidine,

(H)   1-[4-amino-3,5-dibromo-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperidine,

(I)    1-[4-amino-3,5-dibromo-N-[[4-(2,4-dihydro-5-phenyl-3(3H)-oxo-1,2,4-triazol-2-yl)-1-piperidinyl]carbonyl]-D-phenylalanyl]-4-(1-piperidinyl)-piperidine,

(J)    1-[4-amino-3,5-dibromo-N-[[4-(2,4-dihydro-5-phenyl-3(3H)-oxo-1,2,4-triazol-2-yl)-1-piperidinyl]carbonyl]-D-phenylalanyl]-4-(1-methyl-4-piperidinyl)-piperazine,

(K)    1-[4-amino-3,5-dibromo-N-[[4-(3,4-dihydro-2(1H)-oxothieno[3,2-d]pyrimidin-3-yl)-1-piperidinyl]carbonyl]-D-phenylalanyl]-4-(1-piperidinyl)-piperidine,

(L)    1-[4-amino-3,5-dibromo-N-[[4-[1,3-dihydro-4-[3-(trifluoromethyl)phenyl]-2(2H)-oxoimidazol-1-yl]-1-piperidin-yl]carbonyl]-D-phenylalanyl]-4-(1-ethyl-4-piperidinyl)-piperidine,

(M)    1-[4-amino-3,5-dibromo-N-[[4-[3,4-dihydro-2(1H)-oxoquinazolin-3-yl]-1-piperidinyl]carbonyl]-D-phenylalanyl]-4-(1-hexyl-4-piperidinyl)-piperidine,

(N)    1-[4-amino-3,5-dibromo-N-[[4-[3,4-dihydro-2(1H)-oxoquinazolin-3-yl]-1-piperidinyl]carbonyl]-D-phenylalanyl]-4-(1-cyclopropylmethyl-4-piperidinyl)-piperidine,

(O)     1-[N-[[4-[3,4-dihydro-2(1H)-oxoquinazolin-3-yl]-1-piperidinyl]carbonyl]-3-ethenyl-D,L-phenylalanyl]-4-(hexahydro-1H-1-azepinyl)-piperidine,

(P) (R,S)-1-[4-[4-(3,4-dihydro-2(1H)-oxoquinazolin-3-yl)-1-piperidinyl]-2-[(4-hydroxy-3, 5-dimethylphenyl)methyl]-1,4-dioxobutyl]-4-(1-piperidinyl)-piperidine,

(Q)    1-[4-amino-3,5-dibromo-N-[[4-[N-(aminocarbonyl)-N-phenylamino]-1-piperidinyl]carbonyl]-D-phenylalanyl]-4-(1-piperidinyl)-piperidine,

(R)    1-[4-amino-3,5-dibromo-N-[[4-(3,4-dihydro-2(1H)-oxoquinazolin-3-yl)-1-piperidinyl]carbonyl]-D-phenylalanyl]-4-(5-methoxy-4-pyrimidinyl)-piperazine,

(S)    1-[4-amino-3,5-dibromo-N-[[4-(1,1-dioxido-3(4H)-oxo-1,2,4-benzothiadiazin-2-yl)-1-piperidinyl]carbonyl]-D-phenylalanyl]-4-(1-piperidinyl)-piperidine,

(T)    1-[4-amino-3,5-dibromo-N-[[4-[2(1H)-oxoquinolin-3-yl]-1-piperidinyl]carbonyl]-D-phenylalanyl]-4-(1-piperidinyl)-piperidine,

(U)    1-[4-amino-3,5-dibromo-N-[[4-[3,4-dihydro-2(1H)-oxoquinazolin-3-yl]-1-piperidinyl]carbonyl]-D-phenylalanyl]-4-[3-(dimethylamino)propyl]-piperazine,

(V)    1-[4-amino-3,5-dibromo-N-[[4-(3,4-dihydro-2(1H)-oxoquinazolin-3-yl)-1-piperidinyl]carbonyl]-D-phenylalanyl]-4-(4-methyl-1-piperazinyl)-piperidine,

(W)    1-[4-amino-3,5-dibromo-N-[[4-[3,4-dihydro-2(1H)-oxoquinazolin-3-yl]-1-piperidinyl]carbonyl]-D-phenylalanyl]-4-[(1-methyl-4-piperidinyl)carbonyl]-piperazine,

(X)  1-[4-amino-3,5-dibromo-N-[[4-[3,4-dihydro-2(1H)-oxoquinazolin-3-yl]-1-piperidinyl]carbonyl]-D-phenylalanyl]-4-[(1-methyl-4-piperazinyl)carbonyl]-piperazine,

(Y)  1-[4-amino-3,5-dibromo-N-[[4-(3,4-dihydro-2(1H)-oxoquinazolin-3-yl)-1-piperidinyl]carbonyl]-D-phenylalanyl]-4-[4-[4-(dimethylamino)butyl]phenyl]-piperazine,

(Z)  1-[4-amino-3,5-dibromo-N-[[4-(3,4-dihydro-2(1H)-oxoquinazolin-3-yl)-1-piperidinyl]carbonyl]-D-phenylalanyl]-4-[4-(dimethylamino)-1-piperidinyl]-piperidine,

(AA)  1-[$N^2$-[[4-(1,3-dihydro-4-phenyl-2(2H)-oxoimidazol-1-yl)-1-piperidinyl]carbonyl]-N'-methyl-D-tryptyl]-4-(4-methyl-1-piperazinyl)-piperidine,

(AB)  1-[$N^2$-[[4-(1,3-dihydro-4-phenyl-2(2H)-oxoimidazol-1-yl)-1-piperidinyl]carbonyl]-N'-(1,1-dimethylethoxycarbonyl)-D-tryptyl]-4-(1-methyl-4-piperidinyl)-piperidine,

(AC)  (R, S)-1-[4-[4-(3, 4-dihydro-2(1H)-oxoquinazolin-3-yl)-1-piperidinyl]-2-[(3,5-dibromo-4-methylphenyl)methyl]-1,4-dioxobutyl]-4-(4-methyl-1-piperazinyl)-piperidine,

(AD)  (R,S)-1-[4-[4-(3,4-dihydro-2(1H)-oxoquinazolin-3-yl)-1-piperidinyl]-2-[(3,5-dibromo-4-methoxyphenyl)methyl]-1,4-dioxobutyl]-4-(1-methyl-4-piperidinyl)-piperidine,

(AE)  (R,S)-1-[4-[4-(3,4-dihydro-2(1H)-oxoquinazolin-3-yl)-1-piperidinyl]-2-[(3,4-dibromophenyl)methyl]-1,4-dioxobutyl]-4-(4-methyl-1-piperazinyl)-piperidine,

(AF)  1-[$N^2$-[N-[[4-(1,3-dihydro-2(2H)-oxobenzimidazol-1-yl)-1-piperidinyl]carbonyl]-3,5-dibromo-D-tyrosyl]-L-lysyl]-4-(4-pyridinyl)-piperazine,

(AG)  1-[4-amino-3,5-dibromo-N-[[4-(1,3-dihydro-6-hydroxy-2(2H)-oxobenzimidazol-1-yl)-1-piperidinyl]carbonyl]-D-phenylalanyl]-4-(1-piperidinyl)-piperidine,

(AH)  1-[$N^2$-[4-amino-3,5-dibromo-N-[[4-(1,3-dihydro-2(2H)-oxobenzimidazol-1-yl)-1-piperidinyl]carbonyl]-D-phenylalanyl]-$N^6$ ,$N^6$-dimethyl-L-lysyl]-4-(4-pyridinyl)-piperazine,

(AI)  1-[$N^2$-[4-amino-3,5-dibromo-N-[[4-(3,4-dihydro-2(1H)-oxoquinazolin-3-yl)-1-piperidinyl]carbonyl]-D-phe-nylalanyl]-$N^6$,$N^6$-dimethyl-L-lysyl]-4-(4-pyridinyl)-piperazine,

(AJ)  (R, S)-1-(2-(4-amino-3, 5-dibromobenzoyl)-4-[4-(3, 4-dihydro-2(1H)-oxoquinazolin-3-yl)-1-piperidinyl]-4-oxobutyl]-4-(1-piperidinyl)-piperidine,

(AK)  1-[4-amino-3,5-dibromo-N-[[4-(3,4-dihydro-2,2-dioxido-2,1, 3-benzothiadiazin-3-yl)-1-piperidinyl]carbonyl]-D-phenylalanyl]-4-(1-piperidinyl)-piperidine,

(AL)  1-[4-amino-3,5-dibromo-N-[[4-[1,3-dihydro-2(2H)-oxoimidazo[4,5-c]quinolin-3-yl]-1-piperidinyl]carbonyl]-D-phenylalanyl]-4-(1-piperidinyl)carbonyl]-piperidine,

(AM)  1-[4-amino-3,5-dibromo-N-[[4-(3,4-dihydro-2(1H)-oxoquinazolin-3-yl)-1-piperidinyl]carbonyl]-D-phenylalanyl]-4-(1-piperidinyl)-piperidine,

(AN)  1-[$N^2$-[3,5-dibromo-N-[[4-(3,4-dihydro-2(1H)-oxoquinazolin-3-yl)-1-piperidinyl]carbonyl]-D-tyrosyl]-$N^6$,$N^6$-dimethyl-L-lysyl]-4-(4-pyridinyl)-piperazine,

(AO)  1-[4-amino-N-[[4-[4-(3-bromophenyl)-1,3-dihydro-2(2H)-oxoimidazol-1-yl]-1-piperidinyl]carbonyl]-3,5-dibromo-D-phenylalanyl]-4-(1-methyl-4-piperidinyl)-piperidine,

(AP)  1-[4-amino-3,5-dibromo-N-[[4-[1,3-dihydro-4-phenyl-2(2H)-oxoimidazol-1-yl]-1-piperidinyl]carbonyl]-D-phenylalanyl]-4-(4-methyl-1-piperazinyl)-piperidine,

(AQ) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-piperidinyl)-piperidine,

(AR) 1-[4-amino-3, 5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(exo-8-methyl-8-azabicyclo[3,2,1]oct-3-yl)-piperazine,

(AS) 1-[3,5-dibromo-N-[[4-(1,3-dihydro-4-phenyl-2(2H)-oxoimidazol-1-yl)-1-piperidinyl]carbonyl]-D-tyrosyl]-4-(1-piperidinyl)-piperidine,

(AT) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-ethyl-4-piperidinyl)-piperazine,

(AU) 1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(hexahydro-4-methyl-1H-1,4-diazepin-1-yl)piperidine,

(AV) 1-[3,5-dibromo-N-[[4-(3,4-dihydro-2(1H)-oxoquinazolin-3-yl)-1-piperidinyl]carbonyl]-D-tyrosyl]-4-[1-(methylsulphonyl)-4-piperidinyl]-piperidine,

(AW) 1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(1-methyl-4-piperidinyl)-piperidine,

(AX) 1-[3,5-dibromo-N-[[4-[1,3-dihydro-4-[3-(trifluoromethyl)phenyl]-2(2H)-oxoimidazol-1-yl]-1-piperidinyl]carbonyl]-D-tyrosyl]-4-(hexahydro-1H-1-azepinyl)-piperidine,

(AY) 1-[3,5-dibromo-N-[[4-(3,4-dihydro-2(1H)-oxoquinazolin-3-yl)-1-piperidinyl]carbonyl]-D-tyrosyl]-4-(1-methyl-4-piperidinyl)-piperidine,

(AZ) 1-[4-amino-3,5-dibromo-N-[[4-(3,4-dihydro-2(1H)-oxoquinazolin-3-yl)-1-piperidinyl]carbonyl]-D-phenylalanyl]-4-(exo-8-methyl-8-azabicyclo[3,2,1]oct-3-yl)-piperazine,

(BA) 1-[4-amino-3,5-dibromo-N-[[4-(1,3-dihydro-4-phenyl-2(2H)-oxoimidazol-1-yl)-1-piperidinyl]carbonyl]-D-phenyl-alanyl]-4-(1-methyl-4-piperidinyl)-piperazine,

(BB) 1-[3,5-dibromo-N-[[4-[1,3-dihydro-4-[3-(trifluoromethyl)phenyl]-2(2H)-oxoimidazol-1-yl]-1-piperidinyl]carbonyl]-D-tyrosyl]-4-(1-piperidinyl)-piperidine,

(BC) 1-[N[6]-acetyl-N[2]-[3,5-dibromo-N-[[4-(3,4-dihydro-2(1H)-oxoquinazolin-3-yl)-1-piperidinyl]carbonyl]-D-tyrosyl]-L-lysyl]-4-(4-pyridinyl)-piperazine,

(BD) 1-[3,5-dibromo-N-[[4-(1,3-dihydro-4-phenyl-2(2H)-oxoimidazol-1-yl)-1-piperidinyl]carbonyl]-D-tyrosyl]-4-(hexahydro-1H-1-azepinyl)-piperidine,

(BE) 1-[4-amino-3,5-dibromo-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperidine,

(BF) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-methyl-4-piperidinyl)-piperidine,

(BG) 1-[3,5-dibromo-N-[[4-(3,4-dihydro-2(1H)-oxoquinazolin-3-yl)-1-piperidinyl]carbonyl]-D-tyrosyl]-4-[1-(hydroxycarbonylmethyl)-4-piperidinyl]-piperidine,

(BH) 1-[4-amino-3,5-dibromo-N-[[4-[3,4-dihydro-2(1H)-oxoquinazolin-3-yl]-1-piperidinyl]carbonyl]-D-phenylalanyl]-4-(1-methylsulphonyl-4-piperidinyl)-piperidine,

(BI) 1-[3,5-dibromo-N-[[4-(3,4-dihydro-2(1H)-oxoquinazolin-3-yl)-1-piperidinyl]carbonyl]-D-tyrosyl]-4-(4-piperidinyl)-piperidine,

(BJ) 1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(1-ethyl-4-piperidinyl)-piperidine,

(BK)    1-[4-amino-3,5-dibromo-N-[[4-(1,3-dihydro-4-(3-hydroxyphenyl)-2(2H)-oxoimidazol-1-yl)-1-piperidinyl] carbonyl]-D-phenylalanyl]-4-(1-methyl-4-piperidinyl)-piperidine,

(BL)    1-[3,5-dibromo-N-[[4-(3,4-dihydro-2(1H)-oxoquinazolin-3-yl)-1-piperidinyl]carbonyl]-D-tyrosyl]-4-(hexahydro-1H-1-azepinyl)-piperidine,

(BM)  1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(1-piperidinyl)-piperidine,

(BN)    1-[4-amino-3,5-dibromo-N-[[4-[4-(3-bromophenyl)-1,3-dihydro-2(2H)-oxoimidazol-1-yl]-1-piperidinyl] carbonyl]-D-phenylalanyl]-4-(exo-8-methyl-8-azabicyclo[3,2,1]oct-3-yl)-piperazine,

(BO)   1-[4-amino-3,5-dibromo-N-[[4-(3,4-dihydro-2(1H)-oxoquinazolin-3-yl)-1-piperidinyl]carbonyl]-D-phenylalanyl]-4-(1-ethyl-4-piperidinyl)-piperidine,

(BP)   1-[4-amino-3,5-dibromo-N-[[4-(3,4-dihydro-2(1H)-oxoquinazolin-3-yl)-1-piperidinyl]carbonyl]-D-phenylalanyl]-4-(1-ethyl-4-piperidinyl)-piperazine,

(BQ)  1-[4-amino-3, 5-dibromo-N-[[4-[1,3-dihydro-4-(3-methoxyphenyl)-2(2H)-oxoimidazol-1-yl]-1-piperidinyl] carbonyl]-D-phenylalanyl]-4-(exo-8-methyl-8-azabicyclo[3,2,1]oct-3-yl)-piperazine,

(BR)  1-[3,5-dibromo-N-[[4-(3,4-dihydro-2(1H)-oxoquinazolin-3-yl)-1-piperidinyl]carbonyl]-D-tyrosyl]-4-[1-(cyclopropylmethyl)-4-piperidinyl]-piperidine,

(BS)   1-[4-amino-3,5-dibromo-N-[[4-(3,4-dihydro-2(1H)-oxoquinazolin-3-yl)-1-piperidinyl]carbonyl]-D-phenylalanyl]-4-(hexahydro-1H-1-azepinyl)-piperidine,

(BT) 1-[4-amino-3,5-dibromo-N-[[4-(3,4-dihydro-2(1H)-oxoquinazolin-3-yl)-1-piperidinyl]carbonyl]-D-phenylalanyl]-4-(4-piperidinyl)-piperidine,

(BU)   1-[3,5-dibromo-N-[[4-(1,3-dihydro-4-phenyl-2(2H)-oxoimidazol-2-yl)-1-piperidinyl]carbonyl]-D-tyrosyl]-4-(4-pyridinyl)-piperidine,

(BV)     1-[3,5-dibromo-N-[[4-[1,3-dihydro-4-[3-(trifluoromethyl)phenyl]-2(2H)-oxoimidazol-1-yl]-1-piperidinyl] carbonyl]-D-tyrosyl]-4-(1-methyl-4-piperidinyl)-piperazine,

(BW)   1-[N2-[3,5-dibromo-N-[[4-[1,3-dihydro-4-[3-(trifluoromethyl)phenyl]-2(2H)-oxoimidazol-1-yl]-1-piperidinyl]carbonyl]-D-tyrosyl]-L-lysyl]-4-(4-pyridinyl)-piperazine,

(BX)     1-[3,5-dibromo-N-[[4-(1,3-dihydro-4-(3-thienyl)-2(2H)-oxoimidazol-1-yl)-1-piperidinyl]carbonyl]-D-tyrosyl]-4-(1-piperidinyl)-piperidine,

(BY)  1-[4-amino-N-[[4-[4-(3-chlorophenyl)-1,3-dihydro-2(2H)-oxoimidazol-1-yl]-1-piperidinyl]carbonyl]-3,5-dibromo-D-phenylalanyl]-4-(1-methyl-4-piperidinyl)-piperidine,

(BZ)  1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(hexahydro-1H-1-azepinyl)-piperidine,

(CA) 1-[4-amino-3,5-dibromo-N-[[4-[1,3-dihydro-4-[3-(trifluoromethyl)phenyl]-2(2H)-oxoimidazol-1-yl]-1-piperidinyl]carbonyl]-D-phenylalanyl]-4-(1-methyl-4-piperidinyl)-piperazine,

(CB) 1-[4-amino-N-[[4-[4-(3-chlorophenyl)-1,3-dihydro-2(2H)-oxoimidazol-1-yl]-1-piperidinyl]carbonyl]-3,5-dibromo-D-phenylalanyl]-4-(hexahydro-1H-1-azepinyl)-piperidine,

(CC)  1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(4-pyridinyl)-piperazine,

(CD)   1-[3,5-dibromo-N-[[4-(1,3-dihydro-4-phenyl-2(2H)-oxoimidazol-1-yl)-1-piperidinyl]carbonyl]-D-tyrosyl]-4-(1-methyl-4-piperidinyl)-piperidine,

(CE)   1-[4-amino-3,5-dibromo-N-[[4-[1,3-dihydro-4-phenyl-2(2H)-oxoimidazol-1-yl]-1-piperidinyl]carbonyl]-D-phenylalanyl]-4-[4-(1-oxoethyl)phenyl]-piperazine,

(CF)   1-[3,5-dibromo-N-[[4-[3,4-dihydro-2(1H)-oxoquinazolin-3-yl]-1-piperidinyl]carbonyl]-D-tyrosyl]-4-(1-methyl-4-piperidinyl)-piperazine,

(CG) 1-[4-amino-3,5-dibromo-N-[[4-[1,3-dihydro-4-(3-nitrophenyl)-2(2H)-oxoimidazol-1-yl]-1-piperidinyl]carbonyl]-D-phenylalanyl]-4-(1-methyl-4-piperidinyl)-piperidine,

(CH)   1-[4-amino-3,5-dibromo-N-[[4-[3,4-dihydro-2(1H)-oxoquinazolin-3-yl]-1-piperidinyl]carbonyl]-D-phenylalanyl]-4-(1-pyrrolidinyl)-piperidine,

(CI)   1-[4-amino-3,5-dibromo-N-[[4-(1,3-dihydro-4-phenyl-2(2H)-oxoimidazol-1-yl)-1-piperidinyl]carbonyl]-D-phenylalanyl]-4-(hexahydro-1H-1-azepinyl)-piperidine and

(CJ)   1-[4-amino-3,5-dibromo-N-[[4-(1,3-dihydro-4-(3-thienyl)-2(2H)-oxoimidazol-1-yl)-1-piperidinyl]carbonyl]-D-phenylalanyl]-4-(1-methyl-4-piperidinyl)-piperazine,

the tautomers, the diastereomers, the enantiomers, the mixtures thereof and the physiologically acceptable salts thereof.

**2.**   Use according to claim 1, **characterised in that** it is done as a supplement to hormone replacement therapy.

**3.**   Use according to claim 1, **characterised in that** the pharmaceutical composition contains only one active substance.

## Revendications

**1.**   Utilisation d'un antagoniste de CGRP pour la production d'un médicament pour la lutte contre les bouffées de chaleur ménopausiques **caractérisée en ce que** l'antagoniste de CGRP est choisi dans le groupe consistant en :

(A)   1-[$N^2$-[3,5-dibromoo-N-[[4-(3,4-dihydro-2(1H)-oxoquinazolin-3-yl)-1-pipéridinyl]carbonyl]-D-tyrosyl]-L-lysyl]-4-(4-pyridinyl)-pipérazine,

(B)   1-[4-amino-3,5-dibromoo-N-[[4-(2,3,4,5-tétrahydro-2(1H)-oxo-1,3-benzodiazépin-3-yl)-1-pipéridinyl]carbonyl]-D-phénylalanyl]-4-(1-pipéridinyl)-pipéridine,

(C)   1-[$N^2$-[4-amino-3,5-dibromo-N-[[4-(3,4-dihydro-2(1H)-oxoquinazolin-3-yl)-1-pipéridinyl]carbonyl]-D-phénylalanyl]-L-lysyl]-4-(4-pyridinyl)-pipérazine

(D)   1-[$N^2$-[4-amino-3,5-dibrorno-N-[[4-(3,4-dihydro-2(1H)-oxoquinazolin-3-yl)-1-pipéridinyl]carbonyl]-D-phénylalanyl]-L-lysyl]-4-(4-pyridinyl)-pipéridine,

(E)   1-[$N^2$-[3,5-dibromo-N-[[4-(1,3-diltydro-4-phényl-2(2H)-oxoimidazol-1-yl)-1-pipéridinyl]carbonyl]-D-tyrosyl]-L-lysyl]-4-(4-pyridinyl)-pipérazine,

(F)   1-[$N^2$-[4-amino-3,5-dibromo-N-[[4-(1,3-dihydro-4-phényl-2(2H)-oxoimidazol-1-yl)-1-pipéridinyl]carbonyl]-D-phénylalanyl]-L-lysyl]-4-(4-pyridinyl)-pipérazine,

(G)   1-[3,5-dibromo-N-[(4-(3,4-dihydro-2(1H)-oxothiéno[3,4-d]pyrimidin-3-yl)-1-pipéridinyl]carbonyl]-D-tyrosyl]-4-(1-pipéridinyl)-pipéridine,

(H)   1-[4-amino-3,5-dibromo-N-[[4-(2,4-dihydro-5-phényl-3(3H)-oxo-1,2,4-triazol-2-yl)-1-pipéridinyl]carbonyl]-D-phénylalanyl]-4-(1-méthyl-4-pipéridinyl)-pipéridine,

(I)  1-[4-amino-3,5-dibromo-N-[[4-(2,4-dihydro-5-phényl-3(3H)-oxo-1,2,4-triazol-2-yl)-1-pipéridinyl]carbonyl]-D-phénylalanyl]-4-(1-pipéridinyl)-pipéridine,

(J)  1-[4-amino-3,5-dibrorno-N-[[4-(2,4-dihydro-5-phényl-3{3H}-oxo-1,2,4-triazol-2-yl)-1-pipéridinyl]carbonyl]-D-phénylalanyl]-4-(1-méthyl-4-pipéridinyl)-pipérazine,

(K)  1-[4-amino-3,5-dibromo-N-[[4-(3,4-dihydro-2(1H)-oxothiéno[3,2-d]-pyrimidin-3-yl)-1-pipéridinyl]carbonyl]-D-phénylalanyl]-4-(1-pipéridinyl)-pipéridine,

(L)  1-[4-amino-3,5-dibromo-N-[[4-[1,3-dihydro-4-[3-(trifluoroométhyl)phényl]-2(2H)-oxoimidazol-1-yl]-1-pipéridinyl]carbonyl]-D-phénylalanyl]-4-(1-éthyl-4-pipéridinyl)-pipéridine,

(M)  1-[4-amino-3,5-dibromo-N-[[4-[3,4-dihydro-2(1H)-oxoquinazolin-3-yl]-1-pipéridinyl]carbonyl]-D-phénylalanyl]-4-(1-hexyl-4-pipéridinyl)-pipéridine,

(N)  1-[4-amino-3,5-dibromo-N-[[4-[3,4-dihydro-2(1H)-oxoquinazolin-3-yl]-1-pipéridinyl]carbonyl]-D-phénylalanyl]-4-(1-cyclopropylméthyl-4-pipéridinyl)-pipéridine,

(O)  1-[N-[[4-[3,4-dihydro-2(1H)-oxoquinazolin-3-yl]-1-pipéridinyl]carbonyl]-3-éthényl-D,L-phénylalanyl]-4-(hexahydro-1H-1-azépinyl)-pipéridine,

(P)  (R,S)-1-[4-[4-(3,4-dihydro-2(1H)-oxoquinazolin-3-yl)-1-pipéridinyl]-2-[(4-hydroxy-3,5-diméthylphényl)méthyl]-1,4-dioxobutyl]-4-(1-pipéridinyl)-pipéridine,

(Q)  1-[4-amino-3,5-dibromo-N-[[4-[N-(aminocarbonyl)-N-phényl-amino]-1-pipéridinyl]carbonyl]-D-phénylalanyl]-4-(1-pipéridinyl)-pipéridine,

(R)  1-[4-amino-3,5-dibromo-N-[[4-(3,4-dihydro-2(1H)-oxoquinazolin-3-yl)-1-pipéridinyl]carbonyl]-D-phénylalanyl]-4-(5-méthoxy-4-pyrimidinyl)-pipérazine,

(S)  1-[4-amino-3,5-dibromo-N-[[4-(1,1-dioxydo-3(4H)-oxo-1,2,4-benzothiadiazin-2-yl)-1-pipéridinyl]carbonyl]-D-phénylalanyl]-4-{1-pipéridinyl)-pipéridine,

(T)  1-[4-amino-3,5-dibromo-N-[[4-[2(1H)-oxoquinolin-3-yl]-1-pipéridinyl]-carbonyl]-D-phénylalanyl]-4-(1-pipéridinyl)-pipéridine,

(U)  1-[4-amino-3,5-dibromo-N-[[4-[3,4-dihydro-2(1H)-oxoquinazolin-3-yl]-1-pipéridinyl]carbonyl]-D-phénylalanyl]-4-[3-(diméthylamino)propyl]-pipérazine,

(V)  1-[4-amino-3,5-dibromo-N-[[4-[3,4-dihydro-2(1H)-oxoquinazolin-3-yl]-1-pipéridinyl]carbonyl]-D-phénylalanyl]-4-(4-méthyl-1-pipérazinyl)-pipéridine,

(W)  1-[4-amino-3,5-dibromo-N-[[4-[3,4-dihydro-2(1H)-oxoquinazolin-3-yl]-1-pipéridinyl]carbonyl)-D-phénylalanyl]-4-[(1-méthyl-4-pipéridinyl)carbonyl]-pipérazine,

(X)  1-[4-amino-3,5-dibromo-N-[[4-[3,4-dihydro-2(1H)-oxoquinazolin-3-yl]-1-pipéridinyl]carbonyl]-D-phénylalanyl]-4-[(1-méthyl-4-pipérazinyl)carbonyl]-pipérazine,

(Y)  1-[4-amino-3,5-dibromo-N-[[4-(3,4-dihydro-2(1H)-oxoquinazolin-3-yl)-1-pipéridinyl]carbonyl]-D-phénylalanyl]-4-[4-[4-(diméthylamino)butyl]phényl]-pipérazine,

(Z)  1-[4-amino-3,5-dibromo-N-[[4-(3,4-dihydro-2(1H)-oxoquinazolin-3-yl)-1-pipéridinyl]carbonyl]-D-phénylalanyl]-4-[4-(diméthylamino)-1-pipéridinyl]-pipéridine,

(AA)  1-[N$^2$-[[4-(1,3-dihydro-4-phényl-2(2H)-oxoimidazol-1-yl)-1-pipéridinyl]-carbonyl]-N'-méthyl-D-tryptyl]-4-(4-méthyl-1-pipérazinyl)-pipéridine,

(AB) 1-(N$^2$-[[4-(1,3-dihydro-4-phényl-2(2H)-oxoimidazol-1-yl)-1-pipéndinyl]-carbonyl]-N'-(1,1-diméthyléthoxycarbonyl)-D-tryptyl]-4-(1-méthyl-4-pipéridinyl)-pipéridine,

(AC) (R,S)-1-[4-[4-(3,4-dihydro-2(1H)-oxoquinazolin-3-yl)-1-pipéridinyl]-2-[(3,5-dibromo-4-méthylphényl)méthyl]-1,4-dioxobutyl]-4-(4-méthyl-1-pipérazinyl)-pipéridine,

(AD) (R,S)-1-[4-[4-(3,4-dihydro-2(1H)-oxoquinazolin-3-yl)-1-pipéridinyl]-2-[(3,5-dibromo-4-méthoxyphényl)méthyl]-1,4-dioxobutyl]-4-(1-méthyl-4-pipéridinyl)-pipéridine,

(AE) (R,S)-1-[4-[4-(3,4-dihydro-2(1H)-oxoquinazolin-3-yl)-1-pipéridinyl]-2-[(3,4-dibromophényl)méthyl]-1,4-dioxobutyl]-4-(4-méthyl-1-pipérazinyl)-pipéridine,

(AF) 1-[N$^2$-[N-[[4-(1,3-dihydro-2(2H)-oxobenzimidazol-1-yl)-1-pipéridinyl]-carbonyl]-3,5-dibromo-D-tyrosyl]-L-lysyl]-4-(4-pyridinyl)-pipérazine,

(AG) 1-[4-amino-3,5-dibromo-N-[[4-(1,3-dihydro-6-hydroxy-2(2H)-oxobenzimidazol-1-yl)-1-pipéridinyl]carbonyl]-D-phénylalanyl]-4-(1-pipéridinyl)-pipéridine,

(AH) 1-[N$^2$-[4-amino-3,5-dibromo-N-[[4-(1,3-dihydro-2(2H)-oxobenzimidazol-1-yl)-1-pipéridinyl]carbonyl]-D-phénylalanyl]-N$^6$,N$^6$-diméthyl-L-lysyl]-4-(4-pyridinyl)-pipérazine,

(AI) 1-[N$^2$-[4-amino-3,5-dibromo-N-[[4-(3,4-dihydro-2(1H)-oxoquinazolin-3-yl)-1-pipéridinyl]carbonyl]-D-phénylalanyl]-N$^6$,N$^6$-diméthyl-L-lysyl]-4-(4-pyridinyl)-pipérazine,

(AJ) (R,S)-1-[2-(4-amino-3,5-dibromobenzoyl)-4-[4-(3,4-dihydro-2(1H)-oxo-quinazolin-3-yl)-1-pipéridinyl]-4-oxobutyl]-4-(1-pipéridinyl)-pipéridine,

(AK) 1-[4-amino-3,5-dibromo-N-[[4-(3,4-dihydro-2,2-dioxydo-2,1,3-benzothiadiazin-3-yl)-1-pipéridinyl]carbonyl]-D-phénylalanyl]-4-(1-pipéridinyl)-pipéridine,

(AL) 1-[4-amino-3,5-dibromo-N-[[4-[1,3-dihydro-2(2H)-oxoimidazo[4,5-c]-quinolin-3-yl]-1-pipéridinyl]carbonyl]-D-phénylalanyl]-4-(1-pipéridinyl)-carbonyl]-pipéridine,

(AM) 1-[4-amino-3,5-dibromo-N-[[4-(3,4-dihydro-2(1H)-oxoquinazolin-3-yl)-1-pipéridinyl]carbonyl]-D-phénylalanyl]-4-(1-pipéridinyl)-pipéridine,

(AN) 1-[N$^2$-[3,5-dibromo-N-[[4-(3,4-dihydro-2(1H)-oxoquinazolin-3-yl)-1-pipéridinyl]carbonyl]-D-tyrosyl]-N$^6$,N$^6$-diméthyl-L-lysyl]-4-(4-pyridinyl)-pipérazine,

(AO) 1-[4-amino-N-[[4-[4-(3-bromophényl)-1,3-dihydro-2(2H)-oxoimidazol-1-yl]-1-pipéridinyl]carbonyl]-3,5-dibromo-D-phénylalanyl]-4-(1-méthyl-4-pipéridinyl)-pipéridine,

(AP) 1-[4-amino-3,5-dibromo-N-[[4-[1,3-dihydro-4-phényl-2(2H)-oxoimidazol-1-yl]-1-pipéridinyl]carbonyl]-D-phénylalanyl]-4-(4-méthyl-1-pipérazinyl)-pipéridine,

(AQ) 1-[4-amino-3,5-dibromo-N-[[4-[1,3-dihydro-4-[3-(trifluorométhyl)phényl]-2(2H)-oxoimidazol-1-yl]-1-pipéridinyl]carbonyl]-D-phénylalanyl]-4-(1-pipéridinyl)-pipéridine,

(AR) 1-[4-amino-3,5-dibromo-N-[[4-[1,3-dihydro-4-[3-(trifluorométhyl)phényl]-2(2H)-oxoimidazol-1-yl]-1-pipéridinyl]carbonyl]-D-phénylalanyl]-4-(exo-8-méthyl-8-azabicyclo[3,2,1]oct-3-yl)-pipérazine,

(AS) 1-[3,5-dibromo-N-[[4-(1,3-dihydro-4-phényl-2(2H)-oxoimidazol-1-yl)-1-pipéridinyl]carbonyl]-D-tyrosyl]-4-(1-pipéridinyl)-pipéridine,

(AT) 1-[4-amino-3,5-dibromo-N-[[4-[1,3-dihydro-4-[3-(trifluorométhyl)phényl]-2(2H)-oxoimidazol-1-yl]-1-pipéridinyl]carbonyl]-D-phénylalanyl]-4-(1-éthyl-4-pipéridinyl)-pipérazine,

(AU) 1-[4-amino-3,5-dibromo-N-[[4-[1,3-dihydro-4-phényl-2(2H)-oxoimidazol-1-yl]-1-pipéridinyl]carbonyl]-D-phénylalanyl]-4-(hexahydro-4-méthyl-1H-1,4-diazépin-1-yl)pipéridine,

(AV)   1-[3,5-dibromo-N-[[4-(3,4-dihydro-2(1H)-oxoquinazolin-3yl]-1-pipéridinyl]carbonyl]-D-tyrosyl]-4-[1-(méthylsulfonyl)-4-pipéridinyl]-pipéridine,

(AW)  1-[4-amino-3,5-dibromo-N-[[4-(1,3-dihydro-4-phényl-2(2H)-oxoimidazol-1-yl]-1-pipéridinyl]carbonyl]-D-phénylalanyl)-4-(1-méthyl-4-pipéridinyl)-pipéridine,

(AX)     1-[3,5-dibromo-N-[[4-[1,3-dihydro-4-[3-(trifluorométhyl)phényl]-2(2H)-oxoimidazol-1-yl]-1-pipéridinyl]carbonyl]-D-tyrosyl]-4-(hexahydro-1H-1-azépinyl)-pipéridine,

(AY)   1-[3,5-dibromo-N-[[4-(3,4-dihydro-2(1H)-oxoquinazolin-3yl]-1-pipéridinyl]-carbonyl]-D-tyrosyl]-4-(1-méthyl-4-pipéridinyl)-pipéridine,

(AZ)  1-[4-amino-3,5-dibromo-N-[[4-(3,4-dihydro-2(1H)-oxoquinazolin-3-yl]-1-pipéridinyl]carbonyl]-D-phénylalanyl]-4-(exo-8-méthyl-8-azabicyclo[3,2,1]oct-3-yl)-pipérazine,

(BA)   1-[4-ainino-3,5-dibromo-N-[[4-(1,3-dihydro-4-phényl-2(2H)-oxoimidazol-1-yl]-1-pipéridinyl]carbonyl]-D-phénylalanyl]-4-(1-méthyl-4-pipéridinyl)-pipérazine,

(BB)     1-[3,5-dibromo-N-[[4-[1,3-dihydro-4-[3-(trifluorométhyl)phényl]-2(2H)-oxoimidazol-1-yl]-1-pipéridinyl]carbonyl]-D-tyrosyl]-4-(1-pipéridinyl)-pipéridine,

(BC)   1-[N$^6$-acétyl-N$^2$-[3,5-dibromo-N-[[4-(3,4-dihydro-2(1H)oxoquinazolin-3-yl]-1-pipéridinyl]carbonyl]-D-tyrosyl]-L-lysyl]-4-(4-pyridinyl)-pipérazine,

(BD)   1-[3,5-dibromo-N-[[4-(1,3-dihydro-4-phényl-2(2H)-oxoimidazol-1-yl]-1-pipéridinyl]carbonyl]-D-tyrosyl]-4-(hexahydro-1H-1-azépinyl)-pipéridine,

(BE)  1-[4-amino-3,5-dibromo-N-[[4-(1,3-dihydro-4-(3-thiényl)-2(2H)-oxo-imidazol-1-yl]-1-pipéridinyl]carbonyl]-D-phénylalanyl]-4-(1-méthyl-4-pipéridinyl)-pipéridine,

(BF)  1-[4-amino-3,5-dibromo-N-[[4-[1,3-dihydro-4-[3-(trifluorométhyl)phényl]-2(2H)-oxoimidazol-1-yl]-1-pipéridinyl]carbonyl]-D-phénylalanyl]-4-(1-méthyl-4-pipéridinyl)-pipéridine,

(BG)   1-[3,5-dibromo-N-[[4-(3,4-dihydro-2(1H)-oxoquinazolin-3yl]-1-pipéridinyl]carbonyl]-D-tyrosyl]-4-[1-(hydroxycarbonylméthyl)-4-pipéridinyl]-pipéridine,

(BH)  1-[4-amino-3,5-dibromo-N-[[4-[3,4-dihydro-2(1H)-oxoquinazolin-3-yl]-1-pipéridinyl]carbonyl]-D-phénylalanyl]-4-(1-méthylsulfonyl-4-pipéridinyl)-pipéridine,

(BI)1-[3,5-dibromo-N-[[4-(3,4-dihydro-2(1H)-oxoquinazolin-3yl]-1-pipéridinyl)-carbonyl]-D-tyrosyl]-4-(4-pipéridinyl)-pipéridine,

(BJ)   1-[4-amino-3,5-dibromo-N-[[4-(1,3-dihydro-4-phényl-2(2H)-oxoimidazol-1-yl]-1-pipéridinyl]carbonyl]-D-phénylalanyl]-4-(1-éthyl-4-pipéridinyl)-pipéridine,

(BK)   1-[4-amino-3,5-dibromo-N-[[4-(1,3-dihydro-4-(3-hydroxyphényl)-2(2H)-oxoimidazol-1-yl]-1-pipéridinyl]carbonyl]-D-phénylalanyl]-4-(1-méthyl-4-pipéridinyl)-pipéridine,

(BL)   1-[3,5-dibrorno-N-[[4-(3,4-dihydro-2(1H)-oxoquinazolin-3yl]-1-pipéridinyl]-carbonyl]-D-tyrosyl]-4-(hexahydro-1H-1-azépinyl)-pipéridine,

(BM)  1-[4-amino-3,5-dibromo-N-[[4-(1,3-dihydro-4-phényl-2(2H)-oxoimidazol-1-yl]-1-pipéridinyl]carbonyl]-D-phénylalanyl]-4-(1-pipéridinyl)-pipéridine,

(BN)    1-[4-amino-3,5-dibromo-N-[[4-[4-(3-bromophényl)-1,3-dihydro-2(2H)-oxo-imidazol-1-yl]-1-pipéridinyl]carbonyl]-D-phénylalanyl]-4-(exo-8-méthyl-8-aza-bicyclo[3,2,1]oct-3-yl)-pipérazine,

(BO)   1-[4-amino-3,5-dibromo-N-[[4-(3,4-dihydro-2(1H)-oxoquinazolin-3-yl]-1-pipéridinyl]carbonyl]-D-phénylalanyl]-4-(1-éthyl-4-pipéridinyl)-pipéridine,

(BP)   1-[4-amino-3,5-dibromo-N-[[4-(3,4-dihydxo-2(1H)-oxoquinazolin-3-yl)-1-pipéridinyl]carbonyl]-D-phénylalanyl]-4-(1-éthyl-4-pipéridinyl)-pipérazine,

(BQ)   1-[4-amino-3,5-dibromo-N-[[4-[1,3-dihydro-4-(3-méthoxyphényl)-2(2H)-oxoimidazol-1-yl]-1-pipéridinyl]carbonyl]-D-phénylalanyl]-4-(exo-8-méthyl-8-azabicyclo[3,2,1]oct-3-yl)-pipérazine,

(BR)   1-[3,5-dibromo-N-[[4-(3,4-dihydro-2(1H)-oxoquinazolin-3-yl)-1-pipéridinyl]carbonyl]-D-tyrosyl]-4-[1-(cyclopropylméthyl)-4-pipéridinyl]-pipéridine,

(BS)   1-[4-amino-3,5-dibromo-N-[[4-(3,4-dihydro-2(1H)-oxoquinazolin-3-yl)-1-pipéridinyl]carbonyl]-D-phénylalanyl]-4-(hexahydro-1H-1-azépinyl)-pipéridine,

(BT) 1-[4-amino-3,5-dibromo-N-[[4-(3,4-dihydro-2(1H)-oxoquinazolin-3-yl)-1-pipéridinyl]carbonyl]-D-phénylalanyl]-4(4-pipéridinyl)-pipéridine,

(BU)   1-[3,5-dibromo-N-[[4-(1,3-dihydro-4-phényl-2(2H)-oxoimidazol-1-yl)-1-pipéridinyl)carbonyl]-D-tyrosyl]-4-(4-pyridinyl)-pipéridine,

(BV)   1-[3,5-dibromo-N-[[4-[1,3-dihydro-4-[3-(trifluorométhyl)phényl]-2(2H)-oxoimidazol-1-yl]-1-pipéridinyl]carbonyl]-D-tyrosyl]-4-(1-méthyl-4-pipéridinyl)-pipérazine,

(BW)   1-[N$^2$-[3,5-dibromo-N-[[4-[1,3-dihydro-4-[3-(trifluorométhyl)phényl]-2(2H)-oxoimidazol-1-yl]-1-pipéridinyl]carbonyl]-D-tyrosyl]-L-lysyl]-4-(4-pyridinyl)-pipérazine,

(BX)   1-[3,5-dibromo-N-[[4-(1,3-dihydro-4-(3-thiényl)-2(2H)-oxoimidazol-1-yl)-1-pipéridinyl]carbonyl]-D-tyrosyl]-4-(1-pipéridinyl)-pipéridine,

(BY) 1-[4-amino-N-[[4-[4-(3-chlorophényl)-1,3-dihydro-2(2H)-oxoimidazol-1-yl]-1-pipéridinyl]carbonyl]-3,5-dibromo-D-phénylalanyl]-4-(1-méthyl-4-pipéridinyl)-pipéridine,

(BZ) 1-[4-amino-3,5-dibromo-N-[[4-[1,3-dihydro-4-[3-(trifluorométhyl)phényl]-2(2H)-oxoimidazol-1-yl]-1-pipéridinyl)carbonyl]-D-phénylalanyl]-4-(hexahydro-1H-1-azépinyl)-pipéridine,

(CA) 1-[4-amino-3,5-dibromo-N-[[4-[1,3-dihydro-4-[3-(trifluorométhyl)phényl]-2(2H)-oxoimidazol-1-yl]-1-pipéridinyl]carbonyl]-D-phénylalanyl]-4-(1-méthyl-4-pipéridinyl)-pipérazine,

(CB) 1-[4-arnino-N-[[4-[4-(3-chlorophényl)-1,3-dihydro-2(2H)-oxoimidazol-1-yl]-1-pipéridinyl]carbonyl]-3,5-dibromo-D-phénylalanyl]-4-(hexahydro-1H-1-azépinyl)-pipéridine,

(CC) 1-[4-amino-3,5-dibromo-N-[[4-(1,3-dihydro-4-phényl-2(2H)-oxoimidazol-1-yl)-1-pipéridinyl]carbonyl]-D-phénylalanyl]-4(4-pyridinyl)-pipérazine,

(CD)   1-[3,5-dibromo-N-[[4-(1,3-dihydro-4-phényl-2(2H)-oxoimidazol-1-yl)-1-pipéridinyl]carbonyl]-D-tyrosyl]-4-(méthyl-4-pipéridinyl)-pipéridine,

(CE)   1-[4-amino-3,5-dibromo-N-[[4-[1,3-dihydro-4-phényl-2(2H)-oxoimidazol-1-yl]-1-pipéridinyl]carbonyl]-D-phénylalanyl]-4-[4-(1-oxoéthyl)phényl]-pipérazine,

(CF)   1-[3,5-dibromo-N-[[4-[3,4-dihydro-2(1H)-oxoquinazolin-3yl]-1-pipéridinyl]-carbonyl]-D-tyrosyl]-4-(1-méthyl-4-pipéridinyl)-pipérazine,

(CG)   1-[4-amino-3,5-dibromo-N-[[4-[1,3-dihydro-4-(3-nitrophényl)-2(2H)-oxo-imidazol-1-yl]-1-pipéridinyl]carbonyl]-D-phénylalanyl]-4-(1-méthyl-4-pipéridinyl)-pipéridine,

(CH)   1-[4-amino-3,5-dibromo-N-[[4-[3,4-dihydro-2(1H)-oxoquinazolin-3-yl]-1-pipéridinyl]carbonyl]-D-phénylalanyl]-4-(1-pyrrolidinyl)-pipéridine,

(CI)  1-[4-amino-3,5-dibromo-N-[[4-(1,3-dihydro-4-phényl-2(2H)-oxoimidazol-1-yl)-1-pipéridinyl]carbonyl]-D-phénylalanyl]-4-(hexahydro-1H-1-azépinyl)-pipéridine et

(CJ)  1-[4-amino-3,5-dibromo-N-[[4-(1,3-dihydro-4-(3-thiényl)-2(2H)-oxoimidazol-1-yl)-1-pipéridinyl]carbonyl]-D-phénylalanyl]-4-(1-méthyl-4-pipéridinyl)-pipérazine,

leurs tautomères, leurs diastéréoisomères, leurs énantiomères, leurs mélanges et leurs sels physiologiquement acceptables.

**2.**  Utilisation selon la revendication 1 **caractérisée en ce que** celle-ci se déroule en complément d'une thérapie de substitution hormonale.

**3.**  Utilisation selon la revendication 1 **caractérisée en ce que** le médicament ne contient qu'un principe actif.